(19) Europäisches Patentamt
European Patent Office
Office européen des brevets

(11) Veröffentlichungsnummer: **0 114 033**
**B1**

(12) **EUROPÄISCHE PATENTSCHRIFT**

(45) Veröffentlichungstag der Patentschrift: **12.10.88**

(51) Int. Cl.⁴: **C 07 D 209/52,** C 07 D 207/444, C 07 C 103/737, C 07 C 101/447, A 61 K 31/40

(21) Anmeldenummer: **83810594.8**

(22) Anmeldetag: **15.12.83**

(54) **Substituierte Azabicycloalkane, ihre Verwendung, pharmazeutische Präparate, welche diese Verbindungen enthalten, und Verfahren zur Herstellung dieser Verbindungen.**

(30) Priorität: **21.12.82 CH 7450/82**

(43) Veröffentlichungstag der Anmeldung: **25.07.84 Patentblatt 84/30**

(45) Bekanntmachung des Hinweises auf die Patenterteilung: **12.10.88 Patentblatt 88/41**

(84) Benannte Vertragsstaaten: **AT BE CH DE FR GB IT LI LU NL SE**

(56) Entgegenhaltungen:
**DE-A-3 223 463**
**US-A-3 166 571**
**US-A-4 231 935**

(73) Patentinhaber: **CIBA- GEIGY AG, Klybeckstrasse 141, CH- 4002 Basel (CH)**

(72) Erfinder: **Stanek, Jaroslav, Dr., Florastrasse 6, CH- 4127 Birsfelden (CH)**
Erfinder: **Schweizer, Ernst, Dr., Hollenweg 59, CH- 4144 Arlesheim (CH)**

**EP 0 114 033 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Gegenstand der vorliegenden Erfindung sind neue Aminophenyl-substituierte Azabicycloalkane mit wertvollen pharmakologischen Eigenschaften und Salze von diesen Verbindungen, die Verwendung von diesen Stoffen bzw. von pharmazeutischen Präparaten, welche diese Stoffe enthalten, pharmazeutische Präparate und Verfahren zur Herstellung dieser neuen Stoffe, sowie Zwischenprodukte und Verfahren zur Herstellung dieser Zwischenprodukte.

In der Deutschen Offenlegungsschrift 3 223 463 sind als Zwischenprodukte ohne Angabe einer pharmazeutischen Indikation 1-Phenyl-3-azabicyclo[3.1.0]hexan-2,4-dion-Verbindungen beschrieben, die an Phenylring durch Halogen, $C_{1-6}$-Alkylamino oder Di-$C_{1-6}$-alkylamino substituiert sind. Diese Verbindungen werden durch Reduktion in die entsprechenden antidepressiv wirksamen 1-Phenyl-3-azabicyclo[3.1.0]hexan-Verbindungem überführt. Der Anmelder hat sich unter Bezugnahme auf diese ältere, aber nachpublizierte Deutsche Offenlegungsschrift und die äquivalenten Anmeldungen BE-893 707 und AT-377 619 freiwillig eingeschränkt und gesonderte Patentansprüche für die Vertragsstaaten DE und BE beziehungsweise AT vorgelegt.

Die vorliegende Erfindung betrifft substituierte 1-Phenyl-3-azabicyclo[3.1.0]hexan-2-4-dione der Formel

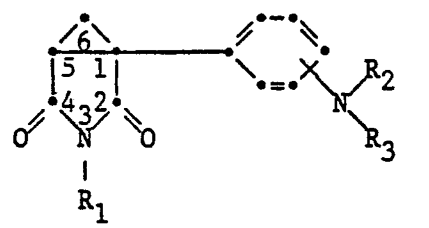 (I),

worin $R_1$ Wasserstoff, $R_2$ Wasserstoff, Sulfo oder Acyl und $R_3$ Wasserstoff oder $R_1$ einen gesättigten oder ungesättigten, aliphatischen, cycloaliphatischen oder cycloaliphatisch-aliphatischen Kohlenwasserstoffrest bis einschliesslich 18, bevorzugt bis einschliesslich 12, C-Atomen, $R_2$ Wasserstoff, Niederalkyl, Sulfo oder Acyl und $R_3$ Wasserstoff oder Niederalkyl bedeuten und Salze dieser Verbindungen.

Die vorliegende Erfindung betrifft ebenfalls pharmazeutische Präparate enthaltend Verbindungen der Formel I, worin $R_1$ Wasserstoff, $R_2$ Wasserstoff oder Niederalkyl und $R_3$ Niederalkyl bedeuten, und die Anwendung dieser Verbindungen in einem Verfahren zur Behandlung des menschlichen und tierischen Körpers.

In der Beschreibung der vorliegenden Erfindung bedeutet der bei der Definition von Gruppen oder Resten, z. B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die in der Beschreibung verwendeten allgemeinen Ausdrücke und Bezeichnungen haben vorzugsweise die folgenden Bedeutungen:

Ein gesättigter oder ungesättigter, aliphatischer, cycloaliphatischer oder cycloaliphatisch-aliphatischer Kohlenwasserstoffrest $R_1$ ist Alkyl, Alkenyl, Niederalkinyl, Cycloalkyl, Cycloalkenyl, Cycloalkylniederalkyl, Cycloalkylniederalkenyl oder Cycloalkenylniederalkyl.

Alkyl $R_1$ hat 1 - 12 C-Atome und ist Niederalkyl mit 1 - 7 C-Atomen, z. B. Methyl, Äthyl, n-Propyl, Isopropyl, n-Butyl, Isobutyl, tert.-Butyl, n-Pentyl, Neopentyl, n-Hexyl oder n-Heptyl, sowie n-Octyl, n-Nonyl, n-Decyl, n-Undecyl oder n-Dodecyl.

Alkenyl $R_1$ hat 2 - 12 C-Atome und ist Niederalkenyl mit 2 - 7 C-Atomen, z. B. Vinyl, Allyl oder 2- oder 3-Butenyl, sowie 1-Octenyl, 1-Nonenyl, 1-Decenyl, 1-Undecenyl oder 1-Dodecenyl, wobei die Doppelbindung sich auch in einer anderen als der 1-Stellung befinden kann.

Niederalkinyl $R_1$ hat z. B. 2 - 7, insbesondere 2 - 4, C-Atome und ist z. B. Äthinyl, 1-Propinyl oder 2-Propinyl.

Cycloalkyl $R_1$ hat z. B. 3 - 10, insbesondere 3 - 6, C-Atome und ist z. B. Cyclopropyl, Cyclobutyl, Cyclopentyl oder Cyclohexyl.

Cycloalkenyl $R_1$ hat z. B. 3 - 10, insbesondere 3 - 6, C-Atome und ist z. B. 1-Cyclohexenyl oder 1,4-Cyclohexadienyl.

Cycloalkylniederalkyl $R_1$ hat z. B. 4 - 10, insbesondere 4 - 7, C-Atome und ist z. B. Cyclopropylmethyl, Cyclobutylmethyl, Cyclopentylmethyl oder Cyclohexylmethyl, sowie 2-Cyclopropyläthyl, 2-Cyclobutyläthyl, 2-Cyclopentyläthyl oder 2-Cyclohexyläthyl.

Cycloalkylniederalkenyl $R_1$ hat z. B. 5 - 10, insbesondere 4 - 9, C-Atome und ist z. B. Cyclohexylvinyl oder Cyclohexylallyl.

Cycloalkenylniederalkyl $R_1$ hat z. B. 4 - 10, insbesondere 4 - 8, C-Atome und ist z. B. 1-Cyclohexenylmethyl oder 1,4-Cyclohexadienylmethyl, sowie 2-(1-Cyclohexenyl)-äthyl oder 2-(1,4-Cyclohexadienyl)-äthyl.

Niederalkyl $R_2$ oder $R_3$ hat die unter $R_1$ genannten Bedeutungen und ist vorzugsweise Methyl oder Äthyl.

Acyl $R_2$ ist vorzugsweise Niederalkanoyl, z. B. Formyl oder Acetyl, oder Niederalkansulfonyl, z. B. Methan- oder Äthansulfonyl.

Salze von erfindungsgemässen Verbindungen der Formel I mit einer salzbildenden Gruppe sind in erster Linie pharmazeutisch verwendbare, nicht-toxische Salze.

Solche Salze werden beispielsweise von der Aminogruppe am Phenylring durch Addition einer anorganischen Säure, z. B. Salzsäure, Schwefelsäure oder Phosphorsäure gebildet und sind beispielsweise, Hydrochloride, Hydrogensulfate, Hydrogenphosphate oder Dihydrogenphosphate.

Weitere Säureadditionssalze werden beispielsweise von Carbonsäuren gebildet und sind beispielsweise Formiate, Acetate, Trifluoracetate, Benzoate oder Salicylate.

Die Verbindungen der Formel 1 können auch als Hydrate vorliegen.

Die vorliegende Erfindung betrifft insbesondere Verbindungen der Formel I, worin $R_1$ Wasserstoff, $R_2$ Wasserstoff, Sulfo, $C_1$-$C_7$-Alkanoyl oder $C_1$-$C_7$-Alkansulfonyl und $R_3$ Wasserstoff oder worin $R_1$ $C_1$-$C_{12}$-Alkyl, $C_2$-$C_{12}$-Alkenyl, $C_2$-$C_7$-Alkinyl, $C_3$-$C_{10}$-Cycloalkyl $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, und Salze dieser Verbindungen, insbesondere pharmazeutisch verwendbare Salze davon.

Die Erfindung betrifft demnach beispielsweise Verbindungen der Formel I, worin $R_1$ Wasserstoff oder $C_1$-$C_7$-Alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, und Salze dieser Verbindungen.

Die vorliegende Erfindung betrifft hauptsächlich Verbindungen der Formel I, worin $R_1$, $R_2$ und $R_3$ Wasserstoff bedeuten oder worin $R_1$ $C_1$-$C_{12}$-Alkyl, z. B. Methyl, Äthyl, n-Propyl, Isopropyl, n-Butyl, Isobutyl, tert.-Butyl, n-Pentyl, Neopentyl, n-Hexyl oder n-Heptyl, sowie n-Octyl, n-Nonyl oder n-Decyl, $C_2$-$C_7$-Alkenyl, z. B. Vinyl oder Allyl, $C_2$-$C_7$-Alkinyl z. B. Äthinyl, 1-Propinyl oder 2-Propinyl, $C_3$-$C_{10}$-Cycloalkyl, z. B. Cyclopropyl, Cyclopentyl oder Cyclohexyl, oder $C_3$-$C_6$-Cycloalkyl-$C_1$-$C_4$-alkyl, z. B. Cyclopentylmethyl oder Cyclohexylmethyl, $R_2$ Wasserstoff oder $C_1$-$C_7$-Alkyl, z. B. Methyl, und $R_3$ Wasserstoff oder $C_1$-$C_7$-Alkyl, z. B. Methyl, bedeuten, sowie Salze davon, insbesondere pharmazeutisch verwendbare Salze davon.

Die vorliegende Erfindung betrifft in erster Linie Verbindungen der Formel I, worin $R_1$ Wasserstoff oder $C_1$-$C_7$-Alkyl, z. B. Methyl, Äthyl, n-Propyl, Isopropyl, n-Butyl, Isobutyl, tert-Butyl, n-Pentyl, Neopentyl, n-Hexyl oder n-Heptyl, und $R_2$ und $R_3$ Wasserstoff bedeuten, sowie Salze davon.

Die vorliegende Erfindung betrifft vor allem Verbindungen der Formel I, worin $R_1$ Wasserstoff $C_1$-$C_7$-Alkyl, z. B. Methyl, Äthyl, n-Propyl, Isopropyl, n-Butyl, Isobutyl oder tert.-Butyl, $C_2$-$C_7$-Alkenyl, z. B. Vinyl oder Allyl, $C_2$-$C_7$-Alkinyl, z. B. Äthinyl oder 1- oder 2-Propinyl, $C_3$-$C_6$-Cycloalkyl-$C_1$-$C_4$-alkyl, z. B. Cyclohexylmethyl, bedeutet, die Gruppe -N($R_2$)($R_3$) sich in 4-Stellung des Phenylrests befindet und $R_2$ und $R_3$ Wasserstoff bedeuten, sowie pharmazeutisch verwendbare Salze davon.

Die Erfindung betrifft demnach beispielsweise Verbindungen der Formel I, worin $R_1$ Wasserstoff oder $C_1$-$C_7$-Alkyl bedeutet, die Gruppe -N($R_2$)($R_3$) sich in 4-Stellung des Phenylrests befindet und $R_2$ und $R_3$ Wasserstoff bedeuten, sowie pharmazeutisch verwendbare Salze davon.

Die vorliegende Erfindung hat vor allem die in den Beispielen genannten Verbindungen zum Gegenstand.

Die neuen Verbindungen der Formel I und ihre pharmazeutisch verwendbaren Salze besitzen beispielsweise als Aromatasehemmer wertvolle pharmakologische Eigenschaften. Die Eignung von Verbindungen der Formel I als Aromatasehemmer kann im "Aromatase Assay" nach Graves P.E. und Salhanick H.A., Endocrinology, Bd. 105, (1979)), S. 52 bei Verwendung von humanen, placentalen Mikrosomen in vitro nachgewiesen werden. In dieser Versuchsanordnung wird die Bildung von Wasser mit Tritiumisotopen und 17β-Östradiol aus [1β,2β-$^3$H]-Testosteron als Folge des Einwirkens einer Verbindung der Formel I auf die Bildung der hydrierten Form des Aromatasecoenzyms Nicotinsäureamid-adenin-di-nucleotidphosphat (NADPH) gemessen. Durch Zugabe einer erfindungsgemässen Verbindung der Formel I, beispielsweise von 1-(4-Aminophenyl)-3-cyclohexylmethyl-3-azabicyclo[3.1.0]hexan-2,4-dion, wird die Enzymaktivität (NADPH-Gehalt) erheblich vermindert, was einen deutlich geringeren Gehalt an Wasser mit radioaktiven Tritiumisotopen als bei Messungen ohne Zugabe einer erfindungsgemässen Verbindung der Formel I zur Folge hat. Vergleichsmessungen zeigen ausserdem, dass die Verringerung der Enzymaktivität als Folge der Zugabe einer erfindunggemässen Verbindung der Formel I, z. B. von 1-(4-Aminophenyl)-3-cyclohexylmethyl-3-azabicyclo[3.1.0]hexan-2,4-dion, bei gleichen Konzentrationen erheblich stärker ist als bei Zugabe von anderen bekannten Aromatasehemmern, z. B. Aminoglutethimid.

Aufgrund ihrer Wirkung als Aromatasehemmer können die Verbindungen der Formel I, worin $R_1$ Wasserstoff, $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, oder Salze davon als Heilmittel, beispielsweise in Form von pharmazeutischen Präparaten, bei der Behandlung von hormonabhängigen Krankheiten, z. B. hormonabhängigen Tumoren, insbesondere Mammakarzinomen, und Anomalien, z. B. Gynäkomastie, bei Warmblütern (Menschen und Tiere) durch enterale, z. B. orale, oder parenterale Applikation von therapeutisch wirksamen Dosen angewendet werden.

Die Anwendung dieser Verbindungen als Heilmittel, insbesondere mit carcinostatischer Wirkung, in einem der genannten Verfahren zur Behandlung des menschlichen oder tierischen Körpers ist ebenfalls Gegenstand der vorliegenden Erfindung.

Die zur Anwendung gelangenden täglichen Dosen von solchen Verbindungen betragen für Säugetiere in Abhängigkeit von der Spezies, sowie für Personen abhängig vom Alter, dem individuellen Zustand und der Applikationsweise zwischen etwa 1 mg und etwa 100 mg, insbesondere zwischen 5 mg und etwa 50 mg, pro kg Körpergewicht. Die Dosen sind innerhalb dieses Bereiches bei parenteraler Applikation, z. B. intramuskulärer oder subcutaner Injektion, oder intravenöser Infusion, im allgemeinen niedriger als bei enteraler, d.h. oraler oder rektaler Applikation. Die Verbindungen der Formel I und pharmazeutisch annehmbaren Salze von solchen

mit salzbildenden Eigenschaften werden oral oder rektal vorzugsweise in Doseneinheitsformen, wie Tabletten, Dragées oder Kapseln bzw. Suppositorien, und parenteral insbesondere als injizierbare Lösungen, Emulsionen oder Suspensionen oder als Infusionslösungen verabreicht, wobei als Lösungen in erster Linie solche von Salzen in Betracht kommen.

Ebenfalls Gegenstand der Erfindung sind pharmazeutische Präparate zur enteralen, z. B. oralen oder rektalen, oder zur parenteralen Verabreichung, welche eine therapeutisch wirksame Menge einer Verbindung der Formel I oder eines pharmazeutisch annehmbaren Salzes einer solchen Verbindung mit einer salzbildenden Gruppe, gegebenenfalls zusammen mit einem pharmazeutisch verwendbaren Trägerstoff oder Trägerstoffgemisch enthalten, wobei als Trägerstoffe feste oder flüssige anorganische oder organische Stoffe verwendet werden. Entsprechende Dosiseinheitsformen, insbesondere zur peroralen Verabreichung, z. B. Dragées, Tabletten oder Kapseln, enthalten vorzugsweise etwa 50 mg bis etwa 500 mg, insbesondere etwa 100 mg bis etwa 400 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Überzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z. B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z. B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z. B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z. B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Öle, z. B. Sesamöl, oder synthetische Fettsäureester, z. B. Äthyloleat, oder Triglyceride verwendet, oder wässrige Injektionssuspensionen, welche viskositätserhöhende Stoffe, z. B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z. B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Ebenfalls Gegenstand der vorliegenden Erfindung sind Verfahren zur Herstellung von Verbindungen der Formel I. Diese können nach an sich bekannten Verfahren hergestellt werden, z. B. indem man

a) in einer Verbindung der Formel

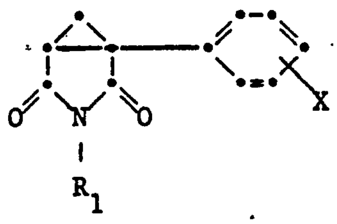

(II),

worin $R_1$ die unter Formel I genannte Bedeutung hat und X eine in die

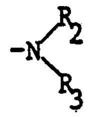

-Gruppe überführbare Gruppe darstellt oder einem Salz davon, X in die

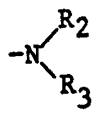

-Gruppe überführt, oder

b) eine Verbindung der Formel

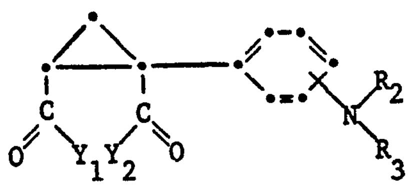

(III)

worin $R_2$ und $R_3$ die unter Formel I genannten Bedeutungen haben, $Y_1$ und $Y_2$ Abgangsgruppen darstellen, oder ein Salz davon durch Umsetzung mit einer die Gruppe

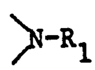

liefernden Verbindung zyklisiert

oder

c) in einer Verbindung der Formel

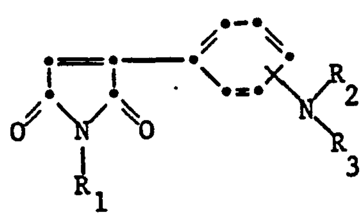

(IV),

worin $R_1$, $R_2$ und $R_3$ die unter Formel I genannte Bedeutung haben, oder in einem Salz davon - $CH_2$ - (Methylen) an die Doppelbindung des Maleimid anlagert und gewünschtenfalls eine erfindungsgemäss erhältliche Verbindung der Formel I in eine andere definitionsgemässe Verbindung der Formel I umwandelt und/oder ein erhältliches Salz in die freie Verbindung oder in ein anderes Salz überführt, und/oder eine erhältliche freie Verbindung in ein Salz überführt und/oder ein erhältliches Gemisch von Isomeren in die einzelnen Isomere auftrennt.

<u>Verfahren a)</u>

In einer Verbindung der Formel II ist eine in die

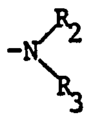

-Gruppe überführbare Gruppe X beispielsweise eine Stickstoff enthaltende reduzierbare Gruppe, z. B. die Nitro-, Nitroso-, Hydroxyamino- oder Azidogruppe, eine austauschbare Gruppe, z. B. Halogen, z. B. Chlor, Brom oder Jod, oder eine derivatisierte Carboxylgruppe, oder eine geschützte Aminogruppe, aus welcher man die Schutzgruppe abspaltet und durch Wasserstoff ersetzt.

Eine Stickstoff enthaltende, reduzierbare Gruppe, z. B. die Nitro-, Nitroso-, Hydroxyamino-, oder Azidogruppe, wird durch ein übliches Reduktionsmittel, welches man gegebenenfalls in Gegenwart eines geeigneten Katalysators und/oder Trägermaterials einsetzt, in die Aminogruppe überführt.

Als übliches Reduktionsmittel kommt in erster Linie in Betracht: Katalytisch aktivierter Wasserstoff, wobei als Hydrierkatalysator beispielsweise ein Edelmetall-, z. B. Palladium-, Platin-, Rhodium- oder Nickelkatalysator, oder eine Edelmetallverbindung, z. B. Platindioxid, verwendet wird und welchen man gegebenenfalls mit einem geeigneten Trägermaterial wie Kohle, Bariumsulfat oder -carbonat oder Calciumcarbonat einsetzt, ein reduzierend wirkendes Zinn(II)- oder Eisen(II)-Salz, die beispielsweise als Chloride und letzteres auch als Sulfat eingesetzt werden, ein reduzierend wirkendes Dithionit- oder Sulfit-Salz, z. B. Natriumdithionit, Natriumsulfit oder Natriumhydrogensulfit, ein unedles, gegebenenfalls aktiviertes Metall, z. B. aktiviertes Eisen, Zinn, Zink oder Aluminium, welches gegebenenfalls in Gegenwart des entsprechenden Metallsalzes oder eines neutralen Salzes, z. B. Calcium-, Magnesium-, Kalium- oder Natriumchlorid aktiviert wird, ferner ein Sulfid, z. B. Schwefelwasserstoff, ein Di- oder Polysulfid, z. B. Natriumdisulfid oder Natriumpolysulfid, ein Alkalimetall- oder Alkalimetallhydrogensulfid, z. B. Natriumsulfid oder Natriumhydrogensulfid, Ammoniumsulfid oder Ammoniumpolysulfid, ein Wasserstoff-abgebendes Reduktionsmittel, z. B. unsubstituiertes oder substituiertes Hydrazin, beispielsweise Hydrazin oder Phenylhydrazin, welches gegebenenfalls in Form eines Säureadditionssalzes, z. B. als Hydrochlorid, zugesetzt wird, oder molekularer Wasserstoff, welcher durch elektrolytische Reduktion an der Kathode entladen wird.

Die Reduktion mit katalytisch aktiviertem Wasserstoff erfolgt bei Normaldruck oder erhöhtem Druck, z. B. bis ca. 6 bar (5 atü). Die Reduktion mit den genannten Reduktionsmitteln erfolgt in saurem, z. B. essigsaurem, oder neutralem Medium. Die Reduktion mit Eisen-(II)-Salzen erfolgt unter basischen Bedingungen, wobei das reduzierend wirkende Eisen-(II)-hydroxid ausfällt. Ebenfalls unter basischen Bedingungen finden die Reduktionen mit Dithionitsalzen und Sulfiden statt.

Die Reduktion mit wasserstoffabgebenden Mitteln z. B. Hydrazinen, wird durch die oben genannten Hydrierkatalysatoren z. B. Raney-Nickel, Palladium-Kohle oder Platin beschleunigt. Die elektrolytische Reduktion der Nitrogruppen zum Amin wird an Kathoden aus Metallen mit grosser Überspannung, wie Blei, Zinn, Nickel, Kupfer oder Zink durchgeführt. Die Elektrolyse erfolgt meist in schwefelsaurer oder salzsaurer Lösung.

Die oben erwähnten Reduktionsmittel werden zumindest in äquimolarer Menge, bevorzugt im Überschuss, zugesetzt. Die Zugabe eines Überschusses an Reduktionsmittel soll die Bildung von Zwischenprodukten, z. B. Nitroso- oder Hydroxyamino-Verbindungen, unterbinden.

Die Reduktion wird vorzugsweise in einem Lösungsmittel durchgeführt, z. B. einem Niederalkanol, z. B. Methanol oder Äthanol, einer Niederalkancarbonsäure oder einem Ester davon, z. B. Essigsäure und Essigsäureäthylester, sowie einem Äther, z. B. Diäthyläther, Tetrahydrofuran oder Dioxan.

Um die Löslichkeit vor allem der salzartigen Reduktionsmittel im Reaktionsgemisch zu erhöhen kann zum Reaktionsgemisch nach Bedarf Wasser hinzugesetzt werden. Man arbeitet gewöhnlicherweise bei Temperaturen von etwa -20°C bis etwa 100°C, wobei bei Verwendung von stark reaktionsfähigen Aktivierungsmitteln die Reaktion auch bei tieferen Temperaturen durchgeführt werden kann.

In einer Verbindung der Formel II wird eine austauschbare Gruppe X, z. B. Halogen, z. B. Chlor, Brom oder Jod, mit einer die Gruppe

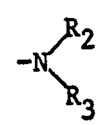

liefernden Verbindung, z. B. Ammoniak, oder einem Alkalimetallamid, z. B. Lithium- oder Natriumamid, einem Niederalkylamin, z. B. Methylamin, einem Diniederalkylamin, z. B. Dimethylamin, einem Säureamid, worin ein Wasserstoff der Amidgruppe durch ein Alkalimetall, z. B. Lithium, ersetzt ist, z. B. $R^a$-CO-$NR_3Li$, in die Gruppe

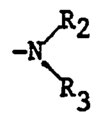

umgewandelt.

Die Umsetzung einer Verbindung der Formel II, worin X Halogen, z. B. Chlor, bedeutet, mit einer die Gruppe

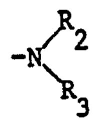

liefernden Verbindung, z. B. mit Ammoniak, erfolgt vorzugsweise in Gegenwart eines Katalysators, z. B. Kupfer-(I)-oxid oder -(II)-oxid, Kupfer-(I)- oder -(II)-chlorid oder Kupfersulfat. Man arbeitet in einer konzentrierten wässrigen Ammoniaklösung oder vorzugsweise in flüssigem Ammoniak und hält die in Houben-Weyl, Methoden der organischen Chemie (im folgenden "Houben-Weyl" genannt), Vol. XI/I "Stickstoffverbindungen", S. 63-67, angegebenen Reaktionsbedingungen ein, z. B. erhöhte Temperatur oberhalb 100° C und erhöhter Druck.

Die Umsetzung einer Verbindung der Formel II, worin X Halogen, z. B. Chlor, bedeutet, erfolgt vorzugsweise mit einem Alkalimetallamid, z. B. Lithium- oder Kaliumamid. Man gibt zweckmässigerweise das Amid in Form einer Suspension hinzu.

Als Lösungsmittel verwendet man vorzugsweise Benzol oder Toluol, und arbeitet unter Inertgas-, z. B. Stickstoffatmosphäre. Die Umsetzung erfolgt am besten bei erhöhter Temperatur, z. B. bei der Siedetemperatur des Reaktionsgemisches, analog den in Houben-Weyl, Vol. X/I "Stickstoffverbindungen" auf S. 74-79 für aromatische Halogenverbindungen beschriebenen Reaktionsbedingungen.

In einer Verbindung der Formel II lässt sich eine derivatisierte Carboxylgruppe X, z. B. Carbamoyl oder Azidocarbonyl, unter den für Umsetzungen bzw. Abbaureaktionen nach Hofmann (Carbamoyl) oder Curtius (Azidocarbonyl) bekannten Reaktionsbedingungen in die Aminogruppe umwandeln.

Die Umwandlung nach Hofmann der Carbamoyl-Verbindung der Formel II mit freiem Halogen, z. B. Brom, in die Aminoverbindung der Formel I erfolgt unter alkalischen Bedingungen.

Die Umwandlung nach Curtius der Azidocarbonyl- (Carbonsäureamid)-Verbindung der Formel II in die Aminoverbindung der Formel I erfolgt thermisch unter Zersetzung der Azidocarbonylgruppe.

Nach der Umlagerung verseift man unter sauren Bedingungen, z. B. in einer verdünnten, wässrigen Mineralsäure, z. B. in verdünnter Schwefelsäure.

Die Reaktionsbedingungen für den Hofmann- und Curtius-Abbau sind in dem Übersichtsartikel von P.A. Smith, Org. Reactions, 3, (1946), 363, beschrieben.

Geschützte Aminogruppen, aus welcher man die Schutzgruppe abspalten und durch Wasserstoff ersetzen kann sind beispielsweise in "Protective Groups in Organic Chemistry", Plenum Press, London und New York 1973, in "The Peptides", Vol. I, Schröder und Lubke, Academic Press, London und New York 1965, sowie in Houben-Weyl, Vol 15/1, Georg Thieme Verlag, Stuttgart 1974, beschrieben.

Als Schutzgruppen sind acidolytisch abspaltbare Gruppen bevorzugt, beispielsweise Niederalkoxycarbonyl, z. B. tert.-Butoxycarbonyl (BOC) oder 2-Halogenniederalkoxycarbonyl, z. B. 2-Jodäthoxy- oder 2,2,2-Trichloräthoxycarbonyl.

Ferner kann man aber auch reduktiv oder unter milden Bedingungen basisch abspaltbare Amino-Schutzgruppen verwenden, z. B. insbesondere die Benzyloxycarbonyl-Gruppe oder eine Benzyloxycarbonyl-Gruppe, worin der Phenylrest durch Halogenatome, Nitrogruppen und/oder Niederalkoxygruppen substituiert ist, z. B. die p-Chlor-, p-Nitro- oder p-Methoxybenzyloxycarbonyl-Gruppe.

Die Abspaltung der Schutzgruppe erfolgt in der allgemein bekannten Weise; die saure Hydrolyse (Acidolyse) wird z. B. mittels Trifluoressigsäure durchgeführt. Die reduktiv abspaltbaren Gruppen, insbesondere solche, die Benzylreste enthalten, werden vorzugsweise hydrogenolytisch, z. B. durch Hydrierung unter Palladium-Katalyse, entfernt.

Verfahren b)

In einer Verbindung der Formel III sind die Abgangsgruppen $Y_1$ und $Y_2$ unabhängig voneinander beispielsweise Hydroxy, Halogen, z. B. Chlor, Brom oder Jod, Niederalkoxy, Silyloxy oder Sulfonyloxy.

Niederalkoxy $Y_1$ oder $Y_2$ ist beispielsweise Methoxy, Äthoxy, n-Propoxy, verzweigtes Niederalkoxy, z. B. tert.-Butoxy, oder substituiertes Niederalkoxy, z. B. Benzyloxy, 4-Nitrobenzyloxy oder Diphenylmethoxy.

Silyloxy $Y_1$ oder $Y_2$ ist beispielsweise Triniederalkylsilyloxy, z. B. Trimethylsilyloxy.

Sulfonyloxy $Y_1$ oder $Y_2$ ist beispielsweise Niederalkansulfonyloxy, z. B. Methansulfonyloxy, Benzolsulfonyloxy oder p-Toluolsulfonyloxy.

Eine die Gruppe

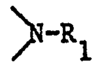

liefernde Verbindung ist beispielsweise ein Alkalimetallamid, z. B. Natrium- oder Kaliumamid, Ammoniak ($R_1$ = Wasserstoff), ein Niederalkylamin, z. B. Methylamin ($R_1$ = Niederalkyl), ein Kohlensäureamid, z. B. Harnstoff oder 1,3-Dimethylharnstoff, oder ein Niederalkancarbonsäureamid, z. B. Formamid, N-Methylformamid, Acetamid oder N-Methylacetamid.

Die Umsetzung mit einer die Gruppe

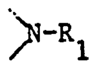

liefernden Verbindung, z. B. mit Ammoniak oder mit Methylamin, kann stufenweise erfolgen. Beispielswise kann man zunächst eine Verbindung der Formel III erhalten, worin eine der Abgangsgruppen $Y_1$ und $Y_2$ durch -NH-$R_1$ ersetzt ist. Eine solche Verbindung, z. B. das Monoamid, lässt sich isolieren oder in situ durch Abspaltung von $HY_1$ oder $HY_2$ in eine Verbindung der Formel I überführen.

Eine Verbindung der Formel III, worin beispielsweise $Y_1$ und $Y_2$ Hydroxy bedeuten, kann man zunächst in ihr Anhydrid überführen, z. B. thermisch oder durch Dehydratisieren mit einem üblichen Dehydratisierungsmittel, z. B. Acetanhydrid oder Acetylchlorid. Dieses Anhydrid kann man isolieren oder in situ durch Umsetzung mit einer die Gruppe

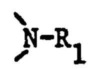

liefernden Verbindung, z. B. mit Ammoniak oder Methylamin, in eine Verbindung überführen, worin eine der Abgangsgruppen $Y_1$ oder $Y_2$ durch -NH-$R_1$ ersetzt ist. Ein solches Monoamid kann man anschliessend durch Abspalten von Wasser zu einer Verbindung der Formel I zyklisieren.

Bei der Zyklisierung werden insgesamt 2 Mol $HY_1$ oder $HY_2$, z. B. HCl oder HBr, freigesetzt, welche beispielsweise von einem eingesetzten Überschuss des

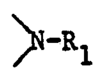

-Lieferanten, z. B. Ammoniak oder Methylamin, gebunden werden.

Die Umsetzung wird bevorzugt in einem inerten polaren Lösungsmittel durchgeführt, z. B. in Benzol, Toluol, Xylol, Methylenchlorid, Äther oder Methanol oder Gemischen davon. Die Reaktionstemperatur liegt zwischen -20°C und ca. +80°C und bevorzugt zwischen 0°C und ca. 30°C. Setzt man ein Säurehalogenid der Formel III, z. B. das Säuredichlorid, mit Ammoniak um, arbeitet man vorzugsweise unter Kühlen, beispielsweise unter 0°C.

Verfahren c)

Die Einführung der $CH_2$-Gruppe in einer Verbindung der Formel IV erfolgt durch Anlagerung einer Methylengruppe an die olefinische Doppelbindung des Maleimids unter Bildung eines Cyclopropylrings. $CH_2$ kann man beispielsweise durch Reaktion mit einer Schwefel-Ylid-Verbindung, z. B. einem (Dialkylamino)-methylsulfoxoniummethylid, z. B. (Dimethylamino)- oder (Diäthylamino)-methylsulfoxoniummethylid, oder einem Dialkylsulfoxoniummethylid, z. B. Dimethyl- oder Diäthylsulfoxoniummethylid, oder mit Dihalogenmethan, z. B. Dichlor-, Dibrom- oder Dijodmethan in Gegenwart eines Zink/Kupfer-Katalysators einführen.

Die betreffende Schwefel-Ylid-Verbindung wird zweckmässigerweise in einem Lösungsmittel hergestellt, siehe beispielsweise C.R. Johnson und P.E. Rogers, J. Org. Chem., Vol 38, No. 10, (1978), S. 1793-1797, Herstellung von (Dimethylamino)-methylsulfoxoniummethylid in Dimethylsulfoxid, und Jzzo P.T., J. Org. Chem., (1963), S. 1713-1715, Herstellung von Dimethylsulfoniummethylid in Tetrahydrofuran, und anschliessend, gegebenenfalls in situ, mit der Verbindung der Formel IV umgesetzt. Die Umsetzung erfolgt normalerweise bei Raumtemperatur, unter Kühlen, z. B. bis auf -20°C, oder unter leichtem Erwärmen, z. B. bis auf 40°C. Das gebildete Dialkylsulfinamid wird bei der anschliessenden wässrigen Aufarbeitung entfernt.

Die Reaktion des Maleimids der Formel IV mit Dihalogenmethan erfolgt unter den für die Simmons-Smith-Reaktion bekannten Reaktionsbedingungen (siehe Houben-Weyl, Vol. 4/3, S. 115-126). Man setzt die

Verbindung der Formel IV mit dem betreffenden Dihalogenmethan, z. B. Dijodmethan, in einem inerten Lösungsmittel um, z. B. in einem Kohlenwasserstoff, z. B. Benzol, einem Äther, z. B. Diäthyläther, oder in einem Ester, z. B. Essigester, in Gegenwart eines Zink/Kupfer-Katalysators, beispielsweise hergestellt aus Zinkstaub und Kupfer-(II)-acetat in Eisessig, und wäscht anschliessend mit Äther. Die Umsetzung erfolgt vorzugsweise bei Raumtemperatur oder leichtem Erwärmen, z. B. oberhalb 30° C.

Die Methylengruppe $-CH_2-$ kann man auch mit Diazomethan einführen welches sich zunächst unter Bildung eines Pyrazolinrings an das Maleinimid anlagern kann. Man vereinigt beispielsweise eine ätherische Lösung von in situ hergestelltem Diazomethan (Herstellung siehe Houben-Weyl, Vol. X/4, S. 473 ff.) mit einer ätherischen Lösung des Maleimids der Fornel IV, wobei sich die Additionsverbindung spontan oder unter leichtem Erwärmen bildet. Die Abspaltung von Stickstoff aus dem Pyrazolinring kann bei Raumtemperatur oder Erwärmen, z. B. bis zur Siedetemperatur des Reaktionsgemisches erfolgen. Durch Zugabe eines Katalysators, z. B. Platin- oder Kupferpulver, kann die Anspaltung von Stickstoff bei Raumtemperatur beschleunigt werden.

Man kann auch Diazomethan in einem inerten Lösungsmittel, z. B. in Hexan oder in Äther, zersetzen, worauf das freie Methylen mit der Maleimid-Verbindung der Formel IV unter direkter Bildung des Cyclopropanrings reagiert. Die Zersetzung von Diazomethan erfolgt vorzugsweise katalytisch, z. B. in Gegenwart eines Edelmetalls in fein verteilter Form, z. B. Kupfer, oder eines Metallsalzes, z. B. Kupfer-(I)-chlorid oder Kupfer-(II)-sulfat.

In Verbindungen der Formel IV, worin $R_1$ Wasserstoff bedeutet, schützt man die Imidofunktion vor der Umsetzung mit Diazomethan mit einer Aminoschutzgruppe. Geeignete Aminoschutzgruppen sind weiter vorn unter Verfahren a) beschrieben.

Statt $CH_2$ kann man auch $C(Hal)_2$, z. B. $C(Cl)_2$, $C(Br)_2$ oder $C(J)_2$, einführen, welches sich an die olefinische Doppelbindung des Maleimids der Formel IV unter Bildung eines Dihalogen-substituierten Cyclopropanrings anlagert. Die Dihalogen-Verbindung lässt sich anschliessend reduktiv in eine Verbindung der Formel I überführen.

Nachoperationen:

In einer Verbindung der Formel I können die Substituenten $R_1$, $R_2$ und $R_3$ im Rahmen ihrer Bedeutungen in andere Substituenten $R_1$, $R_2$ und $R_3$ überführt werden. So kann eine freie Aminogruppe in eine Acylaminogruppe umgewandelt werden, worin $R_2$ Acyl und $R_3$ Wasserstoff oder Niederalkyl bedeuten. Diese nachträglichen Operationen werden in an sich bekannter Weise durchgeführt, beispielsweise wie folgt:

Acylierung der Aminogruppe am Phenylring:

Wenn in einer erhältlichen Verbindung der Formel I $R_2$ und $R_3$ Wasserstoff bedeuten und $R_1$ ein Kohlenwasserstoffrest ist, kann die freie Aminogruppe am Phenylring in an sich bekannter Weise durch eine Acylgruppe $R_2$ oder einen Niederalkylrest $R_3$ substituiert werden. Wenn $R_1$ Wasserstoff ist, ist die Imidogruppe durch eine der weiter vorn genannten Aminoschutzgruppen zu schützen.

Diese Substitution kann beispielsweise durch Acylierung mit einem den entsprechenden Acylrest $R_2$ einführenden geeigneten Acylierungsmittel erfolgen.

Die Aminogruppe am Phenylring liegt in freier Form oder in reaktionsfähiger, d. h. die Acylierung erlaubender, beispielsweise durch Silylreste, geschützter Form vor.

Wird die Aminogruppe am Phenylring in einer Verbindung der Formel I durch einen Acylrest $R^b-SO_2-$ substituiert, verwendet man als Acylierungsmittel beispielsweise die entsprechende Sulfonsäure oder ein reaktionsfähiges, funktionelles Derivat davon, in erster Linie ein Anhydrid davon, z. B. ein gemischtes Anhydrid. Ein gemischtes Anhydrid einer Sulfonsäure wird beispielsweise durch Kondensation mit einer anorganischen Säure, z. B. einer Halogenwasserstoffsäure, z. B. Chlorwasserstoff gebildet und ist beispielsweise das entsprechende Sulfonsäurehalogenid, z. B. das Sulfonsäurechlorid oder -bromid.

Wird die Aminogruppe durch eine Acylgruppe $R^b-CO-$ substituiert, verwendet man als Acylierungsmittel beispielsweise die entsprechende Carbonsäure selbst oder ein reaktionsfähiges, funktionelles Derivat davon.

Ein reaktionsfähiges, d.h. die Carboxamid-Funktion bildendes, funktionelles Derivat einer Carbonsäure ist ein Anhydrid dieser Carbonsäure, vorzugsweise ein gemischtes Anhydrid. Ein gemischtes Anhydrid wird beispielsweise durch Kondensation mit einer anderen Säure, z. B. einer anorganischen Säure, z. B. einer Halogenwasserstoffsäure, gebildet und ist beispielsweise das entsprechende Carbonsäurehalogenid, z. B. das Carbonsäurechlorid oder -bromid. Ein reaktionsfähiges, funktionelles Derivat einer Carbonsäure der Formel III wird ausserdem durch Kondensation mit einem Niederalkylhalbester der Kohlensäure, z. B. dem Äthyl- oder Isobutylhalbester der Kohlensäure gebildet.

Wird die Aminogruppe am Phenylring durch einen Acylrest R mit den Bedeutungen $R^b-O-CO-$, $(R^b)(R^b)N-CO-$ oder $(R^b)(R^b)N-SO_2-$ substituiert, verwendet man als Acylierungsmittel ein reaktionsfähiges Derivat des entsprechenden Kohlensäurehalbesters, der entsprechenden Carbaminsäure oder Amidosulfonsäure. Solche reaktionsfähigen Derivate sind beispielsweise Anhydride, z. B. gemischte Anhydride, welche durch Kondensation mit anorganischen Säuren wie Halogenwasserstoffsäuren, z. B. Chlorwasserstoff, gebildet werden, oder sind bei Verwendung einer Carbaminsäure auch innere Anhydride, z. B. Cyanate.

Die Acylierungsreaktionen werden bevorzugt in Anwesenheit eines geeigneten säurebindenden Mittels, beispielsweise einer geeigneten organischen Base, durchgeführt. Eine geeignete organische Base ist beispielsweise ein Amin, z. B. ein tertiäres Amin, z. B. Triniederalkylamin, z. B. Trimethylamin oder Triäthylamin, ein cyclisches tertiäres Amin, z. B. N-Methylmorpholin, ein bicyclisches Amidin, z. B. ein Diazabicycloalken, z.

B. 1,5-Diazabicyclo[4.3.0]non-5-en oder 1,5-Diazabicyclo[5.4.0]undec-5-en (DBU) oder ist beispielsweise eine Base vom Pyridin-Typ, z. B. Pyridin. Ein geeignetes säurebindendes Mittel ist ferner eine anorganische Base, beispielsweise ein Alkalimetall- oder Erdalkalimetallhydroxid, z. B. Natrium-, Kalium- oder Calciumhydroxid.

Die Acylierungsreaktionen werden vorzugsweise in einem inerten, vorzugsweise wasserfreien, Lösungsmittel oder Lösungsmittelgemisch vorgenommen, beispielsweise in Dimethylformamid, Methylenchlorid, Tetrachlorkohlenstoff, Chlorbenzol, Aceton, Tetrahydrofuran, Essigsäureäthylester, oder Acetonitril, oder in Mischungen davon, gegebenenfalls bei erniedrigter oder erhöhter Temperatur, z. B. in einem Temperaturbereich von etwa -40°C bis etwa +100°C, bevorzugt von etwa -10°C bis etwa +50°C, und gegebenenfalls unter Inertgas-, z. B. Stickstoffatmosphäre.

Die Acylierung der freien Aminogruppe am Phenylring kann sowohl im Endprodukt der Formel I als auch in den Zwischenprodukten der Formeln II, III und IV nach der weiter vorn angegebenen Methode erfolgen.

Substitution der Aminogruppe am Phenylring durch Sulfo:

Wenn in einer erhältlichen Verbindung der Formel I $R_2$ und $R_3$ Wasserstoff bedeuten und $R_1$ ein Kohlenwasserstoffrest ist, kann die freie Aminogruppe am Phenylring in an sich bekannter Weise durch Sulfo substituiert werden.

Dies kann beispielsweise durch Reaktion einer Aminophenylverbindung der Formel I mit Triäthylamin in einem Schwefeltrioxid-Komplex erfolgen.

Damit die Imidofunktion ($R_1$ = Wasserstoff) nicht durch Sulfo substituiert wird, ist diese durch eine der weiter vorn genannten Aminoschutzgruppen zu schützen.

Alkylierung der Aminogruppe am Phenylring:

Wenn in einer erhältlichen Verbindung der Formel I $R_2$ und $R_3$ Wasserstoff bedeuten, kann man die freie Aminogruppe am Phenylring durch zwei Äquivalente eines den Niederalkylrest einführenden, geeigneten Alkylierungsmittels, z. B. eines Alkylhalogenids, z. B. Methylbromid, zur Diniederalkyl-substituierten Aminogruppe ($R_2$ und $R_3$ = Niederalkyl) substituieren.

Die freie Aminogruppe am Phenylring kann man auch durch eine der vorn genannten, üblichen Aminoschutzgruppen, z. B. durch tert.-Butoxycarbonyl, schützen und nach anschliessender Metallierung der so geschützten Aminogruppe mit einem geeigneten Metallierungsreagenz, gefolgt von einer dem Niederalkylrest $R_2$ oder $R_3$ entsprechenden, reaktionsfähigen, alkylierenden Verbindung, alkylieren.

Nach Abspaltung der Aminoschutzgruppen erhält man eine Mononiederalkyl-substituierte Aminogruppe ($R_2$ = H und $R_3$ = Niederalkyl oder $R_2$ = Niederalkyl und $R_3$ = H).

Geeignete Metallierungsreagenzien sind beispielsweise Lithiumdiisopropylamid oder Butyllithium. Eine dem Rest $R_3$ entsprechende reaktionsfähige Verbindung ist beispielsweise eine Verbindung der Formel $R_2$-X oder $R_3$-X, worin X eine nukleofuge Abgangsgruppe, beispielsweise ein Halogenatom, z. B. Chlor, Brom oder Jod, oder eine Sulfonyloxygruppe, z. B. Mesyloxy oder Tosyloxy, darstellt.

Soll die Imidofunktion nicht alkyliert werden ($R_1$ = Wasserstoff), ist diese gegebenenfalls durch eine der weiter vorn genannten üblichen Schutzgruppen zu schützen.

Die Trennung von erfindungsgemässen erhältlichen Diastereomerengemischen in optisch reine Antipoden erfolgt in an sich bekannter Weise, z. B. nach physikalischen oder chemischen Methoden, beispielsweise durch fraktionierte Kristallisation. Man kann aber auch chromatographische Methoden, z. B. Fest-Flüssig-Chromatographie, verwenden. Leichtflüchtige Diastereomerengemische können auch destillativ oder chromatographisch getrennt werden.

Die Trennung von erfindungsgemäss erhältlichen Racematen in optisch reine Antipoden erfolgt in an sich bekannter Weise, z. B. durch Chromatographie an optisch aktive Adsorptionsschichten, beispielsweise an Rohrzucker. Es können auch die Racemate in optisch aktiven Lösungsmitteln gelöst und daraus der schwerer lösliche optische Antipode auskristallisiert werden. Ferner benützt man die unterschiedliche Reaktionsfähigkeit der optischen Antipoden gegenüber biologischem Material wie Mikroorganismen oder isolierten Enzymen, oder man löst die Racemate und kristallisiert einen optischen Antipoden duich Animpfen mit einer kleinen Menge eines nach den obigen Methoden erhaltenen optisch aktiven Produkts aus.

Statt der erfindungsgemäss erhältlichen Verbindung der Formel I selbst kann man auch eine in racemischer Form vorliegende Vorstufe, beispielsweise ein Zwischenprodukt der Formel III, worin $Y_1$ oder $Y_2$ Hydroxy bedeutet, in optisch reine Antipoden auftrennen und dann den optisch reinen Antipoden dieser Vorstufe in einen optisch reinen Antipoden des Endprodukts der Formel I überführen. Die Umsetzungen nach den Verfahren a), b) und d) verändern die Konfiguration des 1- und 5-C-Atoms im allgemeinen nicht. Die Addition von Methylen an die olefinische Doppelbindung des Maleimids der Formel IV gemäss Verfahren c) verläuft im allgemeinen stereospezifisch (cis-Addition).

Man erhält Säureadditionssalze in üblicher Weise, z. B. durch Behandeln mit einer Säure, oder einem geeigneten Anionenaustauscherreagenz.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Ausgangsmaterialien

Die im Verfahren zur Herstellung der Verbindungen der Formel I der vorliegenden Erfindung verwendeten Ausgangsmaterialien sind bekannt oder können, falls sie neu sind, in an sich bekannter Weise hergestellt werden.

Ausgangsmaterialien der Formel II, worin X Halogen oder Nitro bedeutet, sind in der Deutschen Offenlegungsschrift 2 740 562 beschrieben.

Ausgangsmaterialien der Formel II, worin X z. B. Nitroso oder Hydroxyamino, oder eine austauschbare Gruppe, z. B. Carbamoyl oder Azidocarbonyl, oder geschütztes Amino bedeutet, sind neu und ebenfalls Gegenstand der vorliegenden Erfindung. Man kann sie beispielsweise in an sich bekannter Weise herstellen, in dem man eine Verbindung der Formel

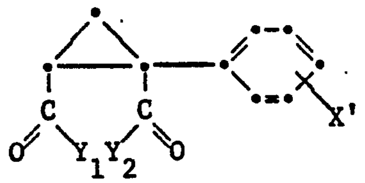

(VI),

worin $Y_1$ und $Y_2$ Abgangsgruppen und X' Nitroso oder Hydroxyamino, Carboxyl oder geschütztes Amino darstellen, oder ein Salz davon durch Umsetzung mit einer die Gruppe -NH-$R_1$ liefernden Verbindung zyklisiert und in einer erhältlichen Verbindung die Carboxylgruppe in eine austauschbare Gruppe, z. B. Carbamoyl oder Azidocarbonyl, überführt und/oder gewünschtenfalls eine erfindungsgemäss erhältliche Verbindung der Formel II in eine andere definitionsgemässe Verbindung der Formel II umwandelt und/oder ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

Das Verfahren lässt sich analog dem weiter vorn unter Verfahren b) genannten Reaktionsbedingungen durchführen. Die Umwandlung der Carboxylgruppe X' in einer Verbindung der Formel II in eine Azidocarbonyl- bzw. Carbamoylgruppe lässt sich anhand der im Organikum, VEB Deutscher Verlag der Wissenschaften, letzte Auflage, für aromatische Carboxylgruppen beschriebenen Derivatisierungsverfahren durchführen.

Ausgangsmaterialien der Formel III sind neu und sind ebenfalls Gegenstand der vorliegenden Erfindung. Man kann sie beispielsweise in an sich bekannter Weise herstellen, indem man eine Verbindung der Formel

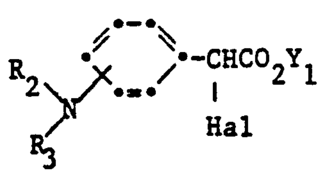

(VII),

worin $R_2$ und $R_3$ die unter Formel I und $Y_1$ die unter Formel III genannte Bedeutung hat und Hal Chlor oder Brom bedeutet, mit einem Acrylsäurederivat der Formel

$HC{=}CHCO_2Y_2$ (VIII),

worin $Y_2$ die unter Formel III genannte Bedeutung hat, umsetzt und gewünschtenfalls ein erhältliches Gemisch von Isomeren in die einzelnen Isomere auftrennt.

Die Herstellung von Verbindungen der Formel III lässt sich analog den von L.L. Mc Coy in J. Am. Chem. Soc., 80, (1958), 6568 und 84, (1962), 2246, sowie in J. Org. Chem. 25, (1960), 2078 genannten Vorschriften durchführen.

Ausgangsmaterialien der Formel IV, worin $R_1$ Wasserstoff und einer der Reste $R_2$ und $R_3$ Acetyl und der andere Wasserstoff bedeutet und deren Herstellung sind in der publizierten japanischen Patentanmeldung 71-35,259, siehe auch CAS Reg. No. 34648-97-0, beschrieben.

Ausgangsmaterialien der Formel IV, worin $R_1$ die unter Formel I genannten Bedeutungen hat, $R_2$ Wasserstoff, Niederalkyl oder Sulfo und $R_3$ Wasserstoff oder Niederalkyl bedeuten, werden z. B. hergestellt, indem man eine Maleimidverbindung der Formel

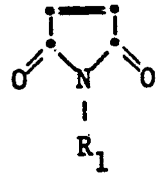

(IX),

worin $R_1$ die unter Formel I genannte Bedeutung hat, mit einem Diazoniumsalz der Formel

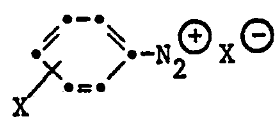

(X),

worin X die unter Formel II genannten Bedeutungen hat und Hal Chlor oder Brom bedeutet, umsetzt und in einer erhältlichen Verbindung X in die Giuppe der Teilformel $-NR_2(R_3)$ überführt und/oder gewünschtenfalls ein erhältliches Salz in die freie Verbindung oder in ein anderes Salz und/oder eine erhältliche freie Verbindung in ein Salz überführt.

Die Umsetzung einer Verbindung der Formel IX mit einem Diazoniumsalz der Formel IX wird analog der Vorschrift von Ch. S. Rondestvedt und O. Vogl in J. Am. Chem. Soc. 77, (1955), 2313-2315 durchgeführt. Die Umwandlung von X in die Gruppe der Teilformel $-NR_2(R_3)$ ist weiter vorn unter Verfahren a) und im Abschnitt "Nachoperationen" beschrieben.

Verbindungen der Formel VI lassen sich analog den Verbindungen der Formel III nach den von L.L. Mc Coy in J. Am. Chem. Soc. 80, (1958), 6258 und 84, (1962), 2246, sowie in J. Org. Chem. 25, (1960), 2078 genannten Vorschriften herstellen.

Ausgangsmaterialien der Formeln VII, VIII, IX und X sind bekannt oder lassen sich, falls sie neu sind, in an sich bekannter Weise herstellen.

Die folgenden Beispiele dienen zur Illustration der Erfindung. Temperaturen sind in Graden Celsius angegeben.

Abkürzungen
Smp. = Schmelzpunkt
Kp. = Siedepunkt

**Beispiel 1:** 1-(4-Aminophenyl)-3-azabicyclo[3.1.0]hexan-2,4-dion

Eine Lösung von 25,9 g 1-(4-Nitrophenyl)-3-azabicyclo[3.1.0]hexan-2,4-dion (Herstellung siehe DE-A-2 740 562) in 700 ml Äthanol wird mit 1,4 g 5-%-igem Palladium/Kohle-Katalysator versetzt und bei Normaldruck und Raumtemperatur in Wasserstoffatmosphäre hydriert. Nach beendeter Wasserstoffaufnahme (theor. 7500 ml) wird das Reaktionsgemisch mit 1 ml Äthanol verdünnt, vom Katalysator befreit und eingedampft. Das so erhältliche Produkt kristallisiert man aus Äthanol um. Man erhält die Titelverbindung mit einem Smp. von 183-185°.

**Beispiel 2:**

a) 1-(3-Aminophenyl)-3-azabicyclo[3.1.0]hexan-2,4-dion

Analog Beispiel 1 werden 600 mg 1-(3-Nitrophenyl)-3-azabicyclo[3.1.0]-hexan-2,4-dion in 30 ml Äthanol gelöst und in Gegenwart von 60 mg 5-%-iger Palladium/Kohle mit Wasserstoff reduziert. Nach beendeter Hydrierung wird das Gemisch mit Äthanol verdünnt und durch Filtration über HYFLO-Super-Cel® vom Katalysator befreit. Man dampft im Vakuum das Lösungsmittel ab, kristallisiert den Rückstand aus einem Essigester-Petroläther-Gemisch um und erhält so die Titelverbindung als blassrosa Kristalle mit einem Smp. 135 - 137° und einem Rf-Wert von 0,47 auf Kieselgeldünnschichtplatten im System Methylenchlorid : Methanol 10 : 1 oder 0,52 im System Methylenchlorid : Methanol : Eisessig 40 : 5 : 1.

Herstellung des Ausgangsmaterials:

b) 2-Chlor-2-(3-nitrophenyl)-essigsäureäthylester

Ein Gemisch von 97 g 3-Nitromandelsäureäthylester und 69 ml Thionylchlorid wird über Nacht bei Raumtemperatur gerührt. Anschliessend werden einige Tropfen Pyridin zugesetzt, und nach Beendigung der Gasentwicklung wird das Gemisch noch ca. 2 Stunden bei 100° gerührt. Man dampft das überschüssige Thionylchlorid im Wasserstrahlvakuum ab und destilliert anschliessend im Hochvakuum. Man erhält die Titelverbindung b) als gelbes Öl mit Kp. 134° bei 0,06 mbar und einem Rf-Wert von 0,5 auf Kieselgeldünnschichtplatten im System Methylenchlorid.

c) 1-(3-Nitrophenyl)-1,2-cyclopropandicarbonsäurediäthylester

Zu einer Suspension von 12,3 g Natriumhydrid (55- bis 60-%-ig im Paraffinöl) in 67 ml Toluol wird unter Rühren und $N_2$-Atmosphäre ein Gemisch von 68,2 g 1-Chlor-1-(3-nitrophenyl)-essigsäureäthylester und 30,5 ml Acrylsäureäthylester bei ca. 50° Innentemperatur des Reaktionsgefässes langsam getropft. Zum Starten und Beschleunigen der Reaktion versetzt man das Reaktionsgemisch gelegentlich mit einigen Tropfen eines Gemisches von Alkohol/Äther 1 : 1. Nach beendeter Reaktion werden vorsichtig 350 ml Wasser zugetropft. Man extrahiert 2 x mit Äther, wäscht die organischen Phasen 2 x mit Wasser und 1 x mit verdünnter Kochsalzlösung. Nach dem Trocknen über Magnesiumsulfat wird filtriert, die Lösung zur Trockene eingedampft und mit Methylenchlorid über 1 kg Kieselgel 0,063 - 0,280 mm chromatographiert. Man erhält so die

Titelverbindung c) als braunes Öl mit einem Rf-Wert von 0,15 auf Kieselgeldünnschichtplatten im System Methylenchlorid.

d) 1-(3-Nitrophenyl)-1,2-cyclopropandicarbonsäure

In 150 ml Methanol werden 14,2 g 1-(3-Nitrophenyl)-1,2-cyclopropandicarbonsäure-diäthylester gelöst und mit 100 ml einer 1,0 N Natronlauge versetzt. Nach dem Rühren über Nacht wird die von der dunkelbraunen Lösung das Lösungsmittel abgezogen. Unter Kühlen auf 0° wird das Gemisch mit 50 ml 2,0 N Salzsäure versetzt und zweimal mit Äther extrahiert. Die Ätherphasen werden mehrmals mit wenig Wasser gewaschen, über Magnesiumsulfat getrocknet, filtriert und eingedampft. Die so erhaltenen Kristalle werden aus siedendem Essigester unter Zugabe von Petroläther umkristallisiert. Man erhält die Titelverbindung d) als blass-beige Kristalle mit Smp. 167 - 170° und Rf = 0,2 auf Kieselgeldünnschichtplatten im System Methylenchlorid : Methanol : Eisessig 40 : 5 : 1.

e) 1-(3-Nitrophenyl)-3-azabicyclo[3.1.0]hexan-2,4-dion

Ein Gemisch von 9,8 g 1-(3-Nitrophenyl)-1,2-cyclopropandicarbonsäure und 4,9 g Harnstoff in 250 ml Xylol (Isomerengemisch) wird ca. 16 Stunden bei einer Badtemperatur von ca. 150° gerührt. Nun dampft man das Xylol im Vakuum ab und verteilt den Rückstand zweimal zwischen Essigester und Wasser. Die organischen Phasen werden vereinigt, über $MgSO_4$ getrocknet, filtriert und im Vakuum eingedampft. Nach Umkristallisation aus Essigester/Petroläther erhält man die Titelverbindung e) als blassgelbe Kristalle mit Smp. 173 - 174° und einem Rf von 0,3 auf Kieselgeldünnschichtplatten im System Hexan : Essigester 4 : 6.

**Beispiel 3:**

a) 1-(4-Aminophenyl)-3-methyl-3-azabicyclo[3.1.0]hexan-2,4-dion

Eine Lösung von 1,5 g 3-Methyl-1-(4-nitrophenyl)-3-azabicyclo[3.1.0]-hexan-2,4-dion in 50 ml Äthanol wird in Gegenwart von 0,1 g 5-%-iger Palladium/Kohle hydriert. Nach beendeter Hydrierung wird der Katalysator abgetrennt, das Filtrat eingedampft und der Rückstand aus Essigester/Petroläther umkristallisiert. Man erhält die Titelverbindung als blassrosa Kristalle mit einem Smp. von 137 - 139° und einem Rf-Wert von 0,47 auf Kieselgeldünnschichtplatten im System Methylenchlorid : Methanol 15 : 1.

Herstellung des Ausgangsmaterials:

b) 3-Methyl-1-(4-nitrophenyl)-3-azabicyclo[3.1.0]hexan-2,4-dion

In 25 ml Dimethylformamid werden 2,32 g 1-(4-Nitrophenyl)3-azabicyclo[3.1.0]hexan-2,4-dion gelöst und unter Stickstoffatmosphäre mit 0,3 g Natriumhydrid (prakt. Fluka) versetzt. Nach 30 Minuten Rühren bei Raumtemperatur wird das Gemisch auf 0° gekühlt und tropfenweise mit einer Lösung von 0,93 ml Methyljodid in 5 ml Dimethylformamid versetzt. Nach weiteren 5 Stunden Rühren wird das überschüssige Natriumhydrid durch Zugabe von Methanol zersetzt. Dann wird im Vakuum das Lösungsmittel abgezogen. Den Rückstand verteilt man zwischen Essigester/Wasser und konzentrierter, wässriger Kochsalzlösung. Die wässrigen Phasen werden einmal mit Essigester gewaschen. Die organischen Phasen werden nach dem Trocknen über Magnesiumsulfat filtriert und eingedampft. Nach Umkristallisieren aus Essigester/Petroläther erhält man die Titelverbindung b) als farblose Kristalle mit einem Smp. 148 - 150° und einem Rf-Wert von 0,7 auf Kieselgeldünnschichtplatten im System Methylenchlorid : Methanol : Eisessig 40 : 5 : 1.

**Beispiel 4:** 3-Äthyl-1-(4-aminophenyl)-3-azabicyclo[3.1.0]hexan-2,4-dion

Analog Beispiel 3 werden 4,1 g 3-Äthyl-1-(4-nitrophenyl)-3-azabicyclo[3.1.0]hexan-2,4-dion in 120 ml Äthanol gelöst, in Gegenwart von 0,2 g 5-%-iger Palladium/Kohle hydriert und aufgearbeitet. Man erhält die Titelverbindung, welche nach Umkristallisation aus Essigester/Petroläther bei 116 - 118° schmilzt.

Das verwendete Ausgangsmaterial wird ausgehend von 4,6 g 1-(4-Nitrophenyl)-3-azabicyclo[3.1.0]hexan-2,4-dion, 0,72 g Natriumhydrid (prakt. Fluka) und 2 ml Äthyljodid analog Beispiel 3 synthetisiert, Smp. 155 - 157° (Essigester/Petroläther).

**Beispiel 5:** 1-(4-Aminophenyl)-3-n-propyl-3-azabicyclo[3.1.0]hexan-2,4-dion

Analog Beispiel 3 werden 3,4 g 1-(4-Nitrophenyl)-3-n-propyl-3-azabicyclo[3.1.0]hexan-2,4-dion in 120 ml Äthanol gelöst, in Gegenwart von 0,2 g 5-%-iger Palladium/Kohle hydriert und aufgearbeitet. Man erhält die Titelverbindung, welche nach Umkristallisation aus Essigester/Petroläther bei 114 - 115° schmilzt.

Das verwendete Ausgangsmaterial wird ausgehend von 4,6 g 1-(4-Nitrophenyl)-3-azabicyclo[3.1.0]hexan-2,4-dion, 0,72 g Natriumhydrid (prakt. Fluka) und 2,44 ml Propyljodid analog Beispiel 3 synthetisiert, Smp. 97° (Äther).

**Beispiel 6:** 1-(4-Aminophenyl)-3-isopropyl-3-azabicyclo[3.1.0]hexan-2,4-dion

Analog Beispiel 3 werden 3,4 g 3-Isopropyl-1-(4-nitrophenyl)-3-azabicyclo[3.1.0]hexan-2,4-dion in 100 ml Äthanol gelöst, in Gegenwart von 0,17 g 5-%-iger Palladium/Kohle hydriert und aufgearbeitet. Man erhält die Titelverbindung, welche nach Umkristallisation aus Essigester/Petroläther bei 160 - 163° schmilzt.
Das verwendete Ausgangsmaterial wird ausgehend von 4,6 g 1-(4-Nitrophenyl)-3-azabicyclo[3.1.0]hexan-2,4-dion, 0,72 g Natriumhydrid (prakt. Fluka) und 2,5 ml Isopropyljodid analog Beispiel 3 synthetisiert, Smp. 115 - 118° (Essigester/Petroläther).

**Beispiel 7:** 1-(4-Aminophenyl)-3-n-butyl-3-azabicyclo[3.1.0]hexan-2,4-dion

Analog Beispiel 3 werden 4,0 g 3-n-Butyl-1-(4-nitrophenyl)-3-azabicyclo[3.1.0]hexan-2,4-dion in 120 ml Äthanol gelöst, in Gegenwart von 0,2 g 5-%-iger Palladium/Kohle hydriert und aufgearbeitet. Man erhält die Titelverbindung, welche nach Umkristallisation aus Äther/Petroläther bei 95 - 98° schmilzt.
Das verwendete Ausgangsmaterial wird ausgehend von 4,6 g 1-(4-Nitrophenyl)-3-azabicyclo[3.1.0]hexan-2,4-dion, 0,72 g Natriumhydrid (prakt. Fluka) und 2,9 ml n-Butyljodid analog Beispiel 3 synthetisiert; gelbes Öl vom Rf-Wert 0,2 auf Kieselgeldünnschichtplatten im System Hexan : Essigester 2 : 1.

**Beispiel 8:** 1-(4-Aminophenyl)-3-isobutyl-3-azabicyclo[3.1.0]hexan-2,4-dion

Analog Beispiel 3 werden 0,55 g 3-Isobutyl-1-(4-Nitrophenyl)-3-azabicyclo[3.1.0]hexan-2,4-dion in 25 ml Äthanol gelöst, in Gegenwart von 0,1 g 5-%-iger Palladium/Kohle hydriert und aufgearbeitet. Man erhält die Titelverbindung, welche nach Umkristallisieren aus Äther/Petroläther bei 115 - 117° schmilzt.
Das verwendete Ausgangsmaterial wird ausgehend von 4,6 g 1-(4-Nitrophenyl)-3-azabicyclo[3.1.0]hexan-2,4-dion, 0,72 g Natriumhydrid (prakt. Fluka) und 3,5 ml Isobutyljodid analog Beispiel 3 synthetisiert, Smp. 100 - 101° (Essigester/Petroläther).

**Beispiel 9:** 1-(4-Aminophenyl)-3-n-pentyl-3-azabicyclo[3.1.0]hexan-2,4-dion

Analog Beispiel 3 werden 5,3 g 1-(4-Nitrophenyl)-3-n-pentyl-3-azabicyclo[3.1.0]hexan-2,4-dion in 150 ml Äthanol gelöst, in Gegenwart von 0,25 g 5-%-iger Palladium/Kohle hydriert und aufgearbeitet. Man erhält die Titelverbindung, welche nach Umkristallisieren aus Essigester/Petroläther bei 113 - 115° schmilzt.
Das verwendete Ausgangsmaterial wird ausgehend von 4,6 g 1-(4-Nitrophenyl)-3-azabicyclo[3.1.0]hexan-2,4-dion, 0,72 g Natriunhydrid (prakt. Fluka) und 3,3 ml Pentyljodid analog Beispiel 3 synthetisiert; gelbes Öl vom Rf-Wert 0,52 auf Kieselgeldünnschichtplatten im System Hexan : Essigester 4 : 6.

**Beispiel 10:** 1-(4-Aminophenyl)-3-neopentyl-3-azabicyclo[3.1.0]hexan-2,4-dion

Analog Beispiel 3 werden 0,85 g 3-Neopentyl-1-(4-nitrophenyl)-3-azabicyclo[3.1.0]hexan-2,4-dion in 30 ml Äthanol gelöst, in Gegenwart von 0,1 g 5-%-iger Palladium/Kohle hydriert und aufgearbeitet. Man erhält die Titelverbindung, welche nach Umkristallisation aus Äther/Petroläther bei 140 - 143° schmilzt.
Das verwendete Ausgangsmaterial wird ausgehend von 4,6 g 1-(4-Nitrophenyl)-3-azabicyclo[3.1.0]hexan-2,4-dion, 0,72 g Natriumhydrid (prakt. Fluka) und 3,3 ml Neopentyljodid analog Beispiel 3 synthetisiert, Smp. 118 - 119° (Äther/Petroläther).

**Beispiel 11:** 1-(4-Aminophenyl)-3-n-heptyl-3-azabicyclo[3.1.0]hexan-2,4-dion

Analog Beispiel 3 werden 5,0 g 3-n-Heptyl-1-(4-nitrophenyl)-3-azabicyclo[3.1.0]hexan-2,4-dion in 100 ml Äthanol gelöst, in Gegenwart von 0,3 g 5-%-iger Palladium/Kohle hydriert und aufgearbeitet. Man erhält die Titelverbindung, welche nach Umkristallisation aus Äther/Petroläther bei 78 - 79° schmilzt.
Das verwendete Ausgangsmaterial wird ausgehend von 4,6 g 1-(4-Nitrophenyl)-3-azabicyclo[3.1.0]hexan-2,4-dion, 0,72 g Natriumhydrid (prakt. Fluka) und 3,9 ml n-Heptylbromid analog Beispiel 3 synthetisiert; gelbes Öl vom Rf-Wert 0,55 auf Kieselgeldünnschichtplatten im System Hexan : Essigester 4 : 6.

**Beispiel 12:** 1-(4-Aminophenyl)-3-cyclohexylmethyl-3-azabicyclo[3.1.0]hexan-2,4-dion

Analog Beispiel 3 werden 0,5 g 3-Cyclohexylmethyl-1-(4-nitrophenyl)-3-azabicyclo[3.1.0]hexan-2,4-dion in 30 ml Äthanol gelöst, in Gegenwart von 0,1 g 5-%-iger Palladium/Kohle mit Wasserstoff hydriert und aufgearbeitet. Man erhält die Titelverbindung, welche nach Umkristallisation aus Essigester/Petroläther bei 125 - 128° schmilzt.

Das verwendete Ausgangsmaterial wird ausgehend von 2,3 g 1-(4-Nitrophenyl)-3-azabicyclo[3.1.0]hexan-2,4-dion, 0,36 g Natriumhydrid (prakt. Fluka) und 1,7 g Cyclohexylmethylchlorid analog Beispiel 3 synthetisiert, Smp. 125 - 128° (Äther/Petroläther).

**Beispiel 13:** 1-(4-Aminophenyl)-3-benzyl-3-azabicyclo[3.1.0]hexan-2,4-dion

Analog Beispiel 3 werden 2,0 g 3-Benzyl-1-(4-nitrophenyl)-3-azabicyclo[3.1.0]hexan-2,4-dion in 100 ml Äthanol gelöst, in Gegenwart von 0,2 g 5-%-iger Palladium/Kohle mit Wasserstoff hydriert und aufgearbeitet. Man erhält die Titelverbindung, welche nach Umkristallisation aus Äthanol bei 154 - 155° schmilzt.

Das verwendete Ausgangsmaterial wird ausgehend von 2,3 g 1-(4-Nitrophenyl)-3-azabicyclo[3.1.0]hexan-2,4-dion, 0,3 g Natriumhydrid (prakt. Fluka) und 1,2 ml Benzylchlorid analog Beispiel 3 synthetisiert, Smp. 147 - 149° (Essigester).

**Beispiel 14:**

a) 1-(4-Aminophenyl)-3-azabicyclo[3.1.0]hexan-2,4-dion

Im Glasbombenrohr werden 2,3 g 1-(4-Aminophenyl)-1,2-cyclopropandicarbonsäure in 80 ml ca. 1 N methanolischer Ammoniaklösung während 7 Tagen bei 100°C gerührt. Das Reaktionsgemisch wird im Vakuum zur Trockene eingedampft und anschliessend mit Hexan : Essigester 4 : 6 über Kieselgel chromatografiert. Nach Umkristallisation aus Äthanol erhält man als weisse Kristalle die Titelverbindung, welche mit der Titelverbindung gemäss Beispiel 1 identisch ist.

Herstellung des Ausgangsmaterials:

b) 1-(4-Aminophenyl)-1,2-cyclopropandicarbonsäure

20 g 1-(4-Nitrophenyl)-1,2-cyclopropandicarbonsäure werden in 200 ml Äthanol gelöst und in Gegenwart von 5-%-iger Palladium/Kohle hydriert. Die erhaltene Suspension wird mit Methanol verdünnt und das ausgefallene Produkt durch Zugabe von 1 Äquivalent methanolischer Salzsäure in Lösung gebracht. Anschliessend wird der Katalysator abfiltriert. Man dampft im Vakuum einen Teil des Lösungsmittels ab, versetzt mit 1 Äquivalent 2,0 N Natronlauge und dampft anschliessend zur Trockne ein. Den erhaltenen Rückstand kristallisiert man aus Wasser um und trocknet diesen im Vakuum über KOH-Pillen bei 60°. Man erhält die Titelverbindung b) als blassbraune Kristalle mit Smp. von über 230° und einem Rf von 0,55 auf Kieselgeldünnschichtplatten im System Methylenchlorid : Methanol : Eisessig 20 : 10 : 2.

**Beispiel 15:** 1-(4-Aminophenyl)-3-methyl-3-azabicyclo[3.1.0]hexan-2,4-dion

Im Glasbombenrohr wird ein Gemisch von 220 mg 1-(4-Aminophenyl)-1,2-cyclopropandicarbonsäure, 10 ml Methanol und 0,26 ml Methylamin (40 %) während sieben Tagen bei 100° gerührt. Man dampft das Gemisch im Vakuum ein und chromatographiert den Rückstand mit Hexan : Essigester 4 : 6 über eine Kieselgelsäule. Man erhält nach Umkristallisation aus Essigester/Petroläther als gelbliche Kristalle die Titelverbindung, welche mit der Titelverbindung gemäss Beispiel 3a) identisch ist.

**Beispiel 16:**

a) 1-(4-Aminophenyl)-3-n-propyl-3-azabicyclo[3.1.0]hexan-2,4-dion

Ein Gemisch von 52 mg 1-(4-Aminophenyl)-1,2-cyclopropandicarbonsäure-2-N-propylamid und 5 ml Xylol (Isomerengemisch) wird 24 Stunden bei 150° gerührt. Nach dem Eindampfen wird der Rückstand in Essigester aufgenommen, über Hyflo-Supercel® filtriert und durch Zusatz von Petroläther kristallisiert. Man erhält die Titelverbindung, welche mit der Titelverbindung gemäss Beispiel 5 identisch ist.

Herstellung des Ausgangsmaterials:

b) 1-(4-Nitrophenyl)-1,2-cyclopropandicarbonsäure-2-N-propylamid

Eine Lösung von 2,5 g 1-(4-Nitrophenyl)-1,2-cyclopropandicarbonsäure in 50 ml Tetrahydrofuran wird mit 2,1 g N,N'-Dicyclohexylcarbodiimid versetzt und 1 Stunde bei Raumtemperatur gerührt. Die Suspension wird bei

Raumtemperatur tropfenweise mit einer Lösung von 0,83 ml Propylamin und 10 ml Tetrahydrofuran versetzt.

Es wird bei Raumtemperatur bis zur vollständigen Umsetzung gerührt. Man dampft das Lösungsmittel ab, versetzt den Rückstand mit Wasser und 1 Äquivalent Natronlauge und filtriert den ausgefallenen Harnstoff ab. Das Filtrat wird mit 1 Äquivalent Salzsäure versetzt und zweimal mit Methylenchlorid extrahiert. Die organischen Phasen werden einmal mit Wasser gewaschen, über Magnesiumsulfat getrocknet, filtriert und eingedampft. Nach Umkristallisation aus Essigester/Äther/Petroläther erhält man die Titelverbindung b) als weisse Kristalle mit Smp. 154 - 155° und einem Rf-Wert von 0,45 auf Kieselgeldünnschichtplattem im System Methylenchlorid : Methanol : Eisessig 40 : 5 : 1.

c) 1-(4-Aminophenyl)-1,2-cyclopropandicarbonsäure-2-N-propylamid

Gelöst in 30 ml Äthanol werden 1,5 g 1-(4-Nitrophenyl)-1,2-cyclopropandicarbonsäure-2-N-propylamid in Gegenwart von 5-%-iger Palladium/Kohle hydriert. Das Reaktionsgemisch wird für die Aufarbeitung 1 : 1 mit Methanol verdünnt, durch Filtration vom Katalysator befreit und anschliessend eingedampft. Nach der Umkristallisation aus Isopropanol erhält man die Titelverbindung c) als blass-beige Kristalle mit einem Smp. 170 - 172° (Zersetzung) und einem Rf-Wert von 0,3 auf Kieselgeldünnschichtplatten im System Methylenchlorid: Methanol : Eisessig 40 : 5 : 1.

**Beispiel 17:** 1-(4-Acetylaminophenyl)-3-azabicyclo[3.1.0]hexan-2,4-dion

Eine Lösung von 4 g 1-(4-Aminophenyl)-3-azabicyclo[3.1.0]hexan-2,4-dion und 120 mg Dimethylaminopyridin in 150 ml Tetrahydrofuran wird tropfenweise mit einer Lösung von 2,4 ml Acetanhydrid in 10 ml Tetrahydrofuran versetzt, und man lässt über Nacht bei Raumtemperatur rühren. Anschliessend wird das Reaktionsgemisch mit 0,5 ml Äthanol versetzt, 1 Stunde gerührt und filtriert. Die Kristalle werden mit Tetrahydrofuran und Äther gewaschen. Man erhält die Titelverbindung als weisse Kristalle mit Smp. oberhalb 230° und einem Rf von 0,3 auf Kieselgeldünnschichtplatten im System Methylenchlorid : Methanol 10 : 1.

**Beispiel 18:** 1-(4-Aminophenyl)-3-azabicyclo[3.1.0]hexan-2,4-dion

61 mg 1-(4-Acetyl-aminophenyl)-3-azabicyclo[3.1.0]hexan-2,4-dion werden während 3 Stunden bei 100° in 0,5 ml ca. 18-%-iger Salzsäure gerührt. Das Reaktionsgemisch wird auf 0° gekühlt und mit gesättigter, wässriger Bicarbonatlösung auf pH 7 gestellt. Der kristalline Niederschlag wird abfiltriert, mit etwas kaltem Wasser nachgewaschen und aus Äthanol umkristallisiert. Man erhält die Titelverbindung, welche mit der Titelverbindung gemäss Beispiel 1 identisch ist.

**Beispiel 19:** 1-(4-Acetylaminophenyl)-3-methyl-3-azabicyclo[3.1.0]hexan-2,4-dion

Unter $N_2$-Atmosphäre werden 1,2 g 1-(4-Acetylaminophenyl)-3-azabicyclo[3.1.0]hexan-2,4-dion, 120 mg Natriumhydrid und 25 ml N,N'-Dimethylformamid während 15 Minuten bei 50° gerührt. Nach Abkühlen auf 20° tropft man eine Lösung von 0,32 ml Methyljodid in 2,5 ml Dimethylformamid langsam zu. Man rührt 16 Stunden bei Raumtemperatur nach. Das überschüssige Natriumhydrid wird mit Methanol zersetzt und das Reaktionsgemisch im Hochvakuum vom Lösungsmittel befreit. Der Rückstand wird mehrmals zwischen Essigester und verdünnter Kochsalzlösung verteilt. Die organischen Phasen werden nach dem Trocknen über Magnesiumsulfat filtriert, eingedampft und aus Essigester/Petroläther kristallisiert. Man erhält die Titelverbindung als weisse Kristalle mit Smp. 183 - 184° und Rf 0,37 auf Kieselgeldünnschichtplatten im System Methylenchlorid : Methanol 10 : 1.

**Beispiel 20:** 1-(4-Aminophenyl)-3-methyl-3-azabicyclo[3.1.0]hexan-2,4-dion

Ein Gemisch von 130 mg 1-(4-Acetylaminophenyl)-3-methyl-3-azabicyclo[3.1.0]hexan-2,4-dion und 1 ml ca. 18-%-iger Salzsäure wird während ca. 4 Stunden bei 100° gerührt. Bei 0° wird das Reaktionsgemisch mit gesättigter, wässriger Sodalösung auf pH 9 - 10 gebracht und zweimal mit Essigester extrahiert. Die organischen Phasen werden mit wenig Wasser, verdünnter Kochsalzlösung neutral gewaschen, über Magnesiumsulfat getrocknet, filtriert und eingedampft. Nach der Umkristallisation aus Essigester/Petroläther erhält man die Titelverbindung, welche mit der Titelverbindung gemäss Beispiel 3a) identisch ist.

**Beispiel 21:** 1-(4-Aminophenyl)-3-methyl-3-azabicyclo[3.1.0]hexan-2,4-dion

Eine Lösung von 202 mg 1-(4-Aminophenyl)-3-azabicyclo[3.1.0]hexan-2,4-dion in 10 ml Methanol wird bei 0° tropfenweise mit 5 ml einer ca. 0,3 molaren Diazomethanlösung versetzt. Das Reaktionsgemisch wird 16 Stunden stehen gelassen, eingedampft und zweimal zwischen Essigester : Wasser verteilt. Die organischen Phasen werden nach dem Trocknen über Magnesiumsulfat filtriert, eingedampft und aus Essigester/Petroläther kristallisiert. Man erhält die Titelverbindung, welche mit der Titelverbindung gemäss Beispiel 3a) identisch ist.

**Beispiel 22:** 1-(4-Methansulfonylaminophenyl)-3-azabicyclo[3.1.0]hexan-2,4-dion

Ein Gemisch von 3 g 1-(4-Aminophenyl)-3-azabicyclo[3.1.0]hexan-2,4-dion, 90 mg Dimethylaminopyridin und 37 ml Pyridin wird bei Raumtemperatur unter Rühren tropfenweise mit einer Lösung von 1,2 ml Methansulfonsäurechlorid in 10 ml Methylenchlorid versetzt. Nach 16 Stunden Rühren wird das Reaktionsgemisch im Vakuum vom Lösungsmittel befreit. Der Rückstand wird in 100 ml Wasser gelöst und mit Salzsäure auf pH 1 gebracht. Man rührt weiter, filtriert die Kristalle ab und kristallisiert diese aus Methanol um. Man erhält die Titelverbindung als weiss-orange Kristalle mit Smp. 208 - 209° und einem Rf-Wert von 0,32 auf Kieselgeldünnschichtplatten im System Methylenchlorid : Methanol 10 : 1.

**Beispiel 23:** 1-(4-Acetylaminophenyl)-3-methyl-3-azabicyclo[3.1.0]hexan-2,4-dion

Eine auf 0° gekühlte Lösung von 2,3 g 3-(4-Acetylaminophenyl)-2,5-dihydro-1H-pyrrol-2,5-dion in 200 ml Tetrahydrofuran wird unter Rühren tropfenweise mit 100 ml einer 0,3 N ätherischen Diazomethanlösung versetzt und 16 Stunden im Eisbad gerührt. Das auskristallisierte Produkt wird abgesaugt, mit Äther nachgewaschen und in 60 ml Äthanol suspendiert. Dieses Gemisch wird 20 Minuten unter Rückfluss erhitzt filtriert und abgekühlt. Man filtriert die weissen Kristalle ab und erhält die Titelverbindung, welche mit der Titelverbindung gemäss Beispiel 19 identisch ist.

**Beispiel 24:** 1-(4-N-Acetyl-N-methylaminophenyl)-3-azabicyclo[3.1.0]hexan-2,4-dion

Ein Gemisch von 3,9 g 1-(4-Methylaminophenyl)-3-azabicyclo[3.1.0]hexan-2,4-dion, 180 ml Tetrahydrofuran, 0,2 g Dimethylaminopyridin und 2 ml Essigsäureanhydrid wird während 20 Stunden bei Raumtemperatur gerührt. Dann wird das Reaktionsgemisch mit 5 ml Äthanol versetzt, weitere 30 Minuten gerührt und anschliessend im Vakuum eingedampft. Der Rückstand wird zweimal zwischen 500 ml Essigester und 50 ml Wasser verteilt. Die vereinigten organischen Phasen werden über Magnesiumsulfat getrocknet, filtriert, eingedampft und aus Essigester kristallisiert. Man erhält die Titelverbindung als weisse Kristalle (Smp. 194 - 195°) und einem Rf-Wert von 0,35 auf Kieselgeldünnschichtplatten im System Methylenchlorid : Methanol 10 : 1.

**Beispiel 25:** 1-(4-N-Acetyl-N-methylaminophenyl)-3-methyl-3-azabicyclo[3.1.0]hexan-2,4-dion

In 40 ml Dimethylformamid werden 1,7 g 1-(4-Acetylmethylaminophenyl)-3-azabicyclo[3.1.0]hexan-2,4-dion gelöst und unter Stickstoffatmosphäre mit 216 mg Natriumhydrid versetzt. Nach 30 Minuten Rühren bei Raumtemperatur wird auf 0° gekühlt und mit 0,9 ml Methyljodid versetzt. Es wird eine Stunde bei 0° und anschliessend 20 Stunden bei Raumtemperatur gerührt. Das überschüssige Natriumhydrid wird durch Zugabe von Methanol zersetzt und das Gemisch anschliessend im Vakuum vom Lösungsmittel befreit. Den Rückstand verteilt man zwischen Essigester/Wasser und verdünnter wässriger Natriumchloridlösung. Man wäscht die wässrigen Phasen einmal mit Essigester. Die vereinigten, über Magnesiumsulfat getrockneten und filtrierten organischen Phasen werden eingedampft. Das erhaltene Öl entgast man während mehreren Stunden im Hochvakuum bei ca. 40°. Man erhält die Titelverbindung als gelbes viskoses Harz mit einem Rf-Wert von 0,36 auf Kieselgeldünnschichtplatten im System Methylenchlorid : Methanol 15 : 1.

**Beispiel 26:** 1-(4-Dimethylaminophenyl)-3-methyl-3-azabicyclo[3.1.0]hexan-2,4-dion und 1-(4-Methylaminophenyl)-3-methyl-3-azabicyclo[3.1.0]hexan-2,4-dion

In 50 ml Dimethylformamid werden 2,16 g 1-(4-Methylaminophenyl)-3-azabicyclo[3.1.0]hexan-2,4-dion gelöst

und unter Stickstoffatmosphäre mit 300 mg Natriumhydrid versetzt. Man rührt 30 Minuten bei Raumtemperatur und tropft anschliessend bei 0° eine Lösung von 0,95 ml Methyljodid in 10 ml Dimethylformamid hinzu. Man rührt das Reaktionsgemisch über Nacht bei Raumtemperatur nach, zersetzt das überschüssige Natriumhydrid mit etwas Methanol und zieht das Lösungsmittel ab. Nach zweimaligem Verteilen des Rückstandes zwischen Essigester und wässriger Natriumchloridlösung werden die organischen Phasen über Magnesiumsulfat getrocknet, filtriert und eingedampft. Der Rückstand wird auf Kieselgel mit Hexan : Essigester 1 : 1 chromatographiert. Man erhält Fraktion I mit Rf-Wert 0,35 und Fraktion II mit Rf-Wert 0,25 auf Kieselgeldünnschichtplatten im System Essigester : Hexan 1 : 1. Nach Umkristallisieren von Fraktion I aus Äther erhält man das 1-(4-Dimethylaminophenyl)-3-methyl-3-azabicyclo[3.1.0]hexan-2,4-dion als weisse Kristalle mit Smp. 140 - 141°. Nach Umkristallisieren der Fraktion II aus Essigester/Petroläther erhält man das 1-(4-Methylaminophenyl)-3-methyl-3-azabicyclo[3.1.0]hexan-2,4-dion als weisse Kristalle mit Smp. 128° - 129°.

**Beispiel 27:** 1-(4-Acetylaminophenyl)-3-allyl-3-azabicyclo[3.1.0]hexan-2,4-dion

In 25 ml Dimethylformamid werden 1,2 g 1-(4-Acetylaminophenyl)-3-azabicyclo[3.1.0]hexan-2,4-dion gelöst und unter Stickstoffatmosphäre mit 180 mg Natriumhydrid versetzt. Es wird 1 Stunde bei Raumtemperatur gerührt. Die klare blassgelbe Lösung wird im Eis-Wasserbad gekühlt und tropfenweise mit einer Lösung von 0,65 ml Allylbromid in 5 ml Dimethylformamid versetzt. Es wird über Nacht gerührt und anschliessend das Lösungsmittel abgezogen. Der Rückstand wird zweimal zwischen Essigester und Wasser verteilt. Die organischen Phasen werden über Magnesiumsulfat getrocknet, filtriert, eingedampft und das Produkt aus Essigester/Äther/Petroläther kristallisiert. Man erhält die Titelverbindung als weisse Kristalle mit Smp. 113 - 116° und einem Rf-Wert von 0,33 auf Kieselgeldünnschichtplatten im System Methylenchlorid : Methanol 10 : 1.

**Beispiel 28:** 1-(4-Aminophenyl)-3-allyl-3-azabicyclo[3.1.0]hexan2,4-dion

Eine Mischung von 2,2 g 1-(4-Acetylaminophenyl)-3-allyl-3-azabicyclo[3.1.0]hexan-2,4-dion, 8 ml Wasser und 8 ml konzentrierter Salzsäure wird während 3 Stunden bei 100° gerührt. Man verdünnt mit etwas Wasser, kühlt im Eiswasserbad und macht das Gemisch mit 30-%-iger Natronlauge alkalisch. Es wird zweimal mit Essigester extrahiert. Die organischen Phasen werden mehrmals mit Wasser und einmal mit wässriger konzentrierter Kochsalzlösung gewaschen, über $MgSO_4$ getrocknet und filtriert. Nach Eindampfen des Filtrats kristallisiert man den Rückstand aus Essigester/Petroläther. Man erhält die Titelverbindung als blassbeige Kristalle mit einem Smp. von 104° - 106° und einem Rf-Wert von 0,57 auf Kieselgeldünnschichtplatten im System Methylenchlorid : Methanol 10 : 1.

**Beispiel 29:** 1-(4-Aminophenyl)-3-propargyl-3-azabicyclo[3.1.0]hexan-2,4-dion

Ein Gemisch von 0,54 g 1-(4-Nitrophenyl)-3-propargyl-3-azabicyclo[3.1.0]hexan-2,4-dion und 1,5 g Zinnpulver in 4 ml Wasser und 4 ml konzentrierter Salzsäure wird eine Stunde bei 100° gerührt. Nach Abkühlung auf Raumtemperatur wird das Reaktionsgemisch mit 20 ml Wasser verdünnt, filtriert und durch Zugabe von Natronlauge alkalisch gemacht. Man extrahiert das Reaktionsgemisch mit Essigester, wäscht die organische Phase mit verdünnter, wässriger Kochsalzlösung neutral, trocknet über Magnesiumsulfat und dampft ein. Der Rückstand wird aus Essigester/Petroläther umkristallisiert. Man erhält die Titelverbindung mit einem Smp. von 106° - 109° und einem Rf-Wert von 0,12 auf Kieselgeldünnschichtplatten im System Hexan : Essigester 1 : 1.

Das verwendete Ausgangsmaterial wird analog Beispiel 3 ausgehend von 4,6 g 1-(4-Nitrophenyl)-3-azabicyclo[3.1.0]hexan-2,4-dion, 0,72 g Natriumhydrid (Fluka, prakt.) und 1,9 ml Propargylbromid in 50 ml N,N-Dimethylformamid synthetisiert. Es schmilzt nach Umkristallisation aus Essigester/Petroläther bei 163 - 165° und hat den Rf-Wert 0,33 auf Kieselgeldünnschichtplatten im System Hexan : Essigester 1 : 1.

**Beispiel 30:** 1-(4-Aminophenyl)-3-n-decyl-3-azabicyclo[3.1.0]hexan-2,4-dion

Analog Beispiel 3 werden 6,0 g 3-n-Decyl-1-(4-nitrophenyl)-3-azabicyclo[3.1.0]hexan-2,4-dion in 120 ml Äthanol gelöst, in Gegenwart von 0,3 g 5-%-iger Palladium/Kohle hydriert und aufgearbeitet. Man erhält die Titelverbindung, welche nach Umkristallisation aus Essigester bei 92 - 94° schmilzt.

Das verwendete Ausgangsmaterial wird ausgehend von 4,6 g 1-(4-Nitrophenyl)-3-azabicyclo[3.1.0]hexan-2,4-dion, 0,72 g Natriumhydrid (prakt. Fluka) und 5,3 ml 1-Decylbromid analog Beispiel 3 synthetisiert; gelbes Öl vom Rf-Wert 0,50 auf Kieselgeldünnschichtplatten im System Hexan : Essigester 1 : 1.

**Beispiel 31:**

a) 1S-5R-1-(4-Aminophenyl)-3-azabicyclo[3.1.0]hexan-2,4-dion

Eine Lösung von 1,3 g 1S-5R-1-(4-Nitrophenyl)-3-azabicyclo[3.1.0]hexan-2,4-dion in 70 ml Äthanol wird analog Beispiel 1 in Gegenwart von 0,1 g 5-%-iger Palladium/Kohle mit Wasserstoff hydriert und aufgearbeitet. Nach Umkristallisation aus Äthanol erhält man die Titelverbindung mit einem Schmelzpunkt von 203° und $[\alpha]^{20}_D$ = -66° ± 1° (Methanol, c = 0,634).

Herstellung des Ausgangsmaterials:

b) 1S-5R-1-(4-Nitrophenyl)-3-azabicyclo[3.1.0]hexan-2,4-dion

Ausgehend von 2,75 g 1S-2R-1-Phenyl-1,2-cyclopropandicarbonsäure ($[\alpha]^{20}_D$ = -193° ± 1° (Methanol, c = 1,09)) wird durch Nitrierung mit 3,45 ml konzentrierter Schwefelsäure und 2 ml Salpetersäure die 1S-2R-1-(4-Nitrophenyl)-1,2-cyclopropandicarbonsäure ($[\alpha]^{20}_D$ = -216° ± 1° (Methanol, c = 0,966)) hergestellt. Dieses Produkt wird in siedendem Xylol in Gegenwart von 1,45 g Harnstoff zur Titelverbindung ($[\alpha]^{20}_D$ = -82° ± 1° (Methanol, c = 0,967)) umgesetzt.

**Beispiel 32:** (-)1S-5R-1-(4-Aminophenyl)-3-azabicyclo[3.1.0]hexan-2,4-dion und (+)1R-5S-1-(4-Aminophenyl)-3-azabicyclo[3.1.0]hexan-2,4-dion

In eine Glassäule werden 380 g Triacetylcellulose, die z. B. nach dem in Chromatographie 6, (1973), 277, beschriebenen Verfahren hergestellt werden kann, in einem Äthanol : Wasser 95 : 5 Gemisch gegeben. Auf diese Säule werden 400 mg 1-(4-Aminophenyl)-3-azabicyclo[3.1.0]hexan-2,4-dion gegeben. Man chromatographiert mit Äthanol/Wasser 95/5 bei einem Druck von 5 - 6 bar und einer Fliessgeschwindigkeit von 350 ml/h. Zuerst wird das mit der Titelverbindung von Beispiel 31a) identische (-) Enantiomere, dann das (+) Enantiomere eluiert. Die Produkte werden separat isoliert, zur Abtrennung von gelöster Triacetylcellulose über Kieselgel filtriert und aus Äthanol umkristallisiert. Man erhält das (-) Enantiomere vom Smp. 202 - 204° und $[\alpha]^{20}_D$ = -65° ± 1° (Methanol, c = 0,5) sowie das (+) Enantiomere vom Smp. 198 - 201° und $[\alpha]^{20}_D$ = -65° ± 1° (Methanol, c = 0,4).

**Beispiel 33:** Lacktabletten, enthaltend 300 mg 1-(4-Aminophenyl)-3-azabicyclo[3.1.0]hexan-2,4-dion, können wie folgt hergestellt werden:

| Zusammensetzung für 10 000 Tabletten | |
|---|---|
| 1-(4-Aminophenyl)-3-azabicyclo[3.1.0]hexan-2,4-dion | 3000,0 g |
| Maisstärke | 680,0 g |
| Kolloidale Kieselsäure | 200,0 g |
| Magnesiumstearat | 20,0 g |
| Stearinsäure | 50,0 g |
| Natriumcarboxymethylstärke | 250,0 g |
| Wasser | q.s. |

Ein Gemisch des 1-(4-Aminophenyl)-3-azabicyclo[3.1.0]hexan-2,4-dion, 50 g Maisstärke und der kolloidalen Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45° während 30 Minuten im Wirbelschichttrockner getrocknet. Das trockene Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Die Presslinge werden in einem Dragierkessel von 45 cm Durchmesser mit einer Lösung von 20 g Schellack und 40 g Hydroxypropylmethylcellulose (niedere Viskosität) in 110 g Methanol und 1350 g Methylenchlorid durch gleichmässiges Aufsprühen während 30 Minuten überzogen; dabei wird durch gleichzeitiges Einblasen von Luft von 60° getrocknet.

Anstelle des oben genannten Wirkstoffes kann man auch dieselbe Menge eines anderen Wirkstoffes der vorangehenden Beispiele verwenden.

**Patentansprüche** (für die Vertragsstaaten FR, IT, LU, NL, SE, GB, CH/LI).

1. Verbindungen der Formel

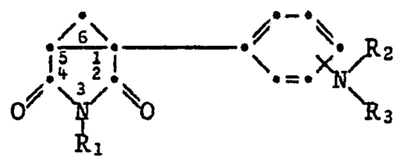

(I),

worin $R_1$ Wasserstoff, $R_2$ Wasserstoff, Sulfo, $C_1$-$C_7$-Alkanoyl oder $C_1$-$C_7$-Alkansulfonyl und $R_3$ Wasserstoff oder worin $R_1$ $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, und Salze dieser Verbindungen.

2. Verbindungen gemäss Anspruch 1 der Formel I, worin $R_1$ Wasserstoff oder $C_1$-$C_7$-Alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, und Salze dieser Verbindungen.

3. Verbindungen gemäss Anspruch 1 der Formel I, worin $R_1$, $R_2$ und $R_3$ Wasserstoff bedeuten oder worin $R_1$ $C_1$-$C_{12}$-Alkyl, $C_2$-$C_7$-Alkenyl, $C_2$-$C_7$-Alkinyl, $C_3$-$C_{10}$-Cycloalkyl oder $C_3$-$C_6$-Cycloalkyl-$C_1$-$C_4$-alkyl, $R_2$ Wasserstoff oder $C_1$-$C_7$-Alkyl und $R_3$ Wasserstoff oder $C_1$-$C_7$-Alkyl bedeuten, sowie Salze davon.

4. Verbindungen gemäss Anspruch 2 der Formel I, worin $R_1$ Wasserstoff oder $C_1$-$C_7$-Alkyl und $R_2$ und $R_3$ Wasserstoff bedeuten, sowie Salze davon.

5. Verbindungen gemäss Anspruch 1 der Formel I, worin $R_1$ Wasserstoff, $C_1$-$C_7$-Alkyl, $C_2$-$C_4$-Alkenyl, $C_2$-$C_4$-Alkinyl oder $C_3$-$C_6$-Cycloalkyl-$C_1$-$C_4$-alkyl bedeutet, die Gruppe -N($R_2$)($R_3$) sich in 4-Stellung des Phenylrests befindet und $R_2$ und $R_3$ Wasserstoff bedeuten, sowie pharmazeutisch verwendbare Salze davon.

6. Verbindungen gemäss Anspruch 2 der Formel I, worin $R_1$ Wasserstoff oder $C_1$-$C_7$-Alkyl bedeutet, die Gruppe -N($R_2$)($R_3$) sich in 4-Stellung des Phenylrests befindet und $R_2$ und $R_3$ Wasserstoff bedeuten, sowie pharmazeutisch verwendbare Salze davon.

7. 1-(4-Aminophenyl)-3-methyl-3-azabicyclo[3.1.0]hexan-2,4-dion gemäss Anspruch 1.

8. 1-(4-Aminophenyl)-3-n-propyl-3-azabicyclo[3.1.0]hexan-2,4-dion gemäss Anspruch 1.

9. 1-(4-Aminophenyl)-3-isobutyl-3-azabicyclo[3.1.0]hexan-2,4-dion gemäss Anspruch 1.

10. 1-(4-Aminophenyl)-3-n-butyl-3-azabicyclo[3.1.0]hexan-2,4-dion gemäss Anspruch 1.

11. 1-(4-Aminophenyl)-3-n-pentyl-3-azabicyclo[3.1.0]hexan-2,4-dion gemäss Anspruch 1.

12. 1-(4-Aminophenyl)-3-neopentyl-3-azabicyclo[3.1.0]hexan-2,4-dion gemäss Anspruch 1.

13. 1-(4-Aminophenyl)-3-n-heptyl-3-azabicyclo[3.1.0]hexan-2,4-dion gemäss Anspruch 1.

14. 1-(4-Aminophenyl)-3-cyclohexylmethyl-3-azabicyclo[3.1.0]hexan-2,4-dion gemäss Anspruch 1.

15. Pharmazeutische Präparate enthaltend Verbindungen der Formel I, worin $R_1$ Wasserstoff, $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl,. $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, oder Salze davon zusammen mit einem pharmazeutisch verwendbaren Trägerstoff.

16. Pharmazeutische Präparate gemäss Anspruch 15 enthaltend 1-(4-Aminophenyl)-3-azabicyclo[3.1.0]hexan-2,4-dion.

17. Verbindungen der Formel I, worin $R_1$, $R_2$ und $R_3$ die in Anspruch 15 genannten Bedeutungen haben, oder Salze davon zur Anwendung in einem Verfahren zur therapeutischen Behandlung des menschlichen oder tierischen Körpers.

18. Verwendung von Verbindungen der Formel I, worin $R_1$, $R_2$ und $R_3$ die in Anspruch 15 genannten Bedeutungen haben, oder von Salzen davon zur Herstellung eines carcinostatischen Heilmittels.

19. Verfahren zur Herstellung von Verbindungen der Formel I gemäss Anspruch 1, und von Salzen dieser Verbindungen, dadurch gekennzeichnet, dass man

a) in einer Verbindung der Formel

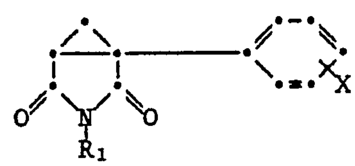

(II),

worin $R_1$ die unter Formel I genannte Bedeutung hat und X eine in die -N($R_2$)($R_3$)-Gruppe überführbare Gruppe darstellt, oder in einem Salz davon X in die -N($R_2$)($R_3$)-Gruppe überführt, oder

b) eine Verbindung der Formel

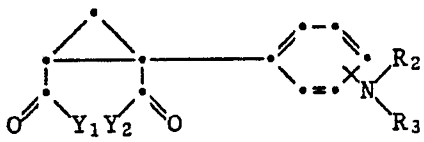 (III),

worin $R_2$ und $R_3$ die unter Formel I genannten Bedeutungen haben, $Y_1$ und $Y_2$ Abgangsgruppen darstellen, oder ein Salz davon durch Umsetzung mit einer die Gruppe

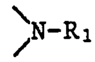

liefernden Verbindung zyklisiert,
oder
c) in einer Verbindung der Formel

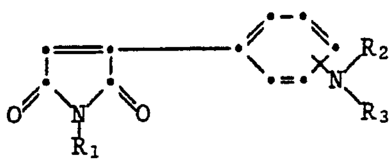 (IV),

worin $R_1$, $R_2$ und $R_3$ die unter Formel I genannte Bedeutung haben, oder in einem Salz davon $-CH_2-$ (Methylen) an die Doppelbindung des Maleimids anlagert, und

gewünschtenfalls eine erfindungsgemäss erhältliche Verbindung der Formel I in eine andere definitionsgemässe Verbindung der Formel I umwandelt und/oder ein erhältliches Salz in die freie Verbindung oder in ein anderes Salz überführt, und/oder eine erhältliche freie Verbindung in ein Salz überführt und/oder ein erhältliches Gemisch von Isomeren in die einzelnen Isomere auftrennt.

20. Verbindungen der Formel

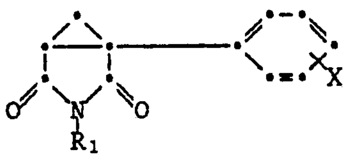 (II),

worin $R_1$ Wasserstoff, $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl und X Nitroso, Hydroxyamino, Carbamoyl, Azidocarbonyl oder geschütztes Amino bedeuten, und Salze von diesen Verbindungen.

21. Verfahren zur Herstellung von Verbindungen der Formel II gemäss Anspruch 20, und von Salzen dieser Verbindungen, dadurch gekennzeichnet, dass man eine Verbindung der Formel

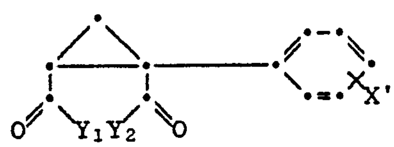 (VI),

worin $Y_1$ und $Y_2$ Abgangsgruppen und X' Nitroso, Hydroxyamino, Carboxyl oder geschütztes Amino darstellen, oder ein Salz davon durch Umsetzung mit einer die Gruppe $-NH-R_1$ liefernden Verbindung zyklisiert und in einer erhältlichen Verbindung die Carboxylgruppe X' in Carbamoyl oder Azidocarbonyl überführt und/oder gewünschtenfalls eine erfindungsgemäss erhältliche Verbindung der Formel II in eine andere definitionsgemässe Verbindung der Formel II umwandelt und/oder ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

22. Verbindungen der Formel

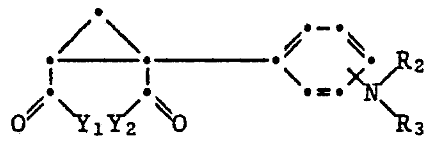

(III),

worin $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten und $Y_1$ und $Y_2$ Abgangsgruppen darstellen, und Salze von diesen Verbindungen mit salzbildenden Gruppen.

23. Verfahren zur Herstellung von Verbindungen der Formel III gemäss Anspruch 22, und von Salzen dieser Verbindungen, dadurch gekennzeichnet, dass man eine Verbindung der Formel

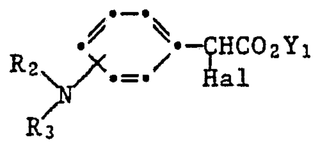

(VII),

worin $R_2$, $R_3$ und $Y_1$ die in Anspruch 22 genannten Bedeutungen haben und Hal Chlor oder Brom bedeutet, mit einem Acrylsäurederivat der Formel

$HC{=}CHCO_2Y_2$ (VIII),

worin $Y_2$ die in Anspruch 22 genannte Bedeutung hat, umsetzt und gewünschtenfalls ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

24. Verfahren zur Herstellung von pharmazeutischen Präparaten enthaltend Verbindungen der Formel I, worin $R_1$, $R_2$ und $R_3$ die in Anspruch 15 genannten Bedeutungen haben, oder Salze davon, dadurch gekennzeichnet, dass man Verbindungen der Formel I und Salze davon nach dem Verfahren gemäss Anspruch 19 herstellt und diese mit einem pharmazeutischen Trägermaterial mischt.

**Patentansprüche** (für den Vertragsstaat AT)

1. Verfahren zur Herstellung von Verbindungen der Formel

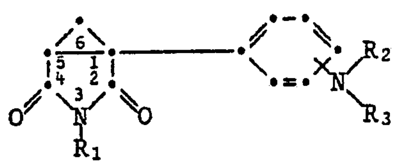

(I),

worin $R_1$ $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, und von Salzen dieser Verbindungen, dadurch gekennzeichnet, dass man

a) in einer Verbindung der Formel

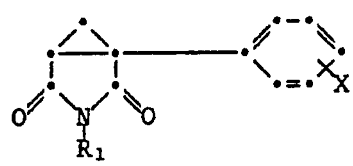

(II),

worin $R_1$ die unter Formel I genannte Bedeutung hat und X eine in die -N($R_2$)($R_3$)-Gruppe überführbare Gruppe darstellt, oder in einem Salz davon X in die -N($R_2$)($R_3$)-Gruppe überführt, oder

b) eine Verbindung der Formel

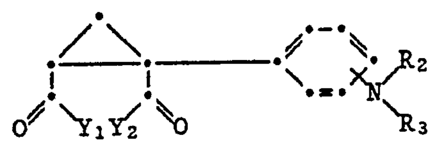

(III),

worin $R_2$ und $R_3$ die unter Formel I genannten Bedeutungen haben, $Y_1$ und $Y_2$ Abgangsgruppen darstellen, oder ein Salz davon durch Umsetzung mit einer die Gruppe

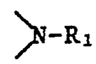

liefernden Verbindung zyklisiert,

oder

c) in einer Verbindung der Formel

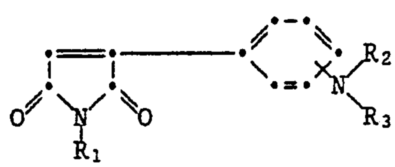

(IV),

worin $R_1$, $R_2$ und $R_3$ die unter Formel I genannte Bedeutung haben, oder in einem Salz davon $-CH_2-$ (Methylen) an die Doppelbindung des Maleimids anlagert, und

gewünschtenfalls eine erfindungsgemäss erhältliche Verbindung der Formel I in eine andere definitionsgemässe Verbindung der Formel I umwandelt und/oder ein erhältliches Salz in die freie Verbindung oder in ein anderes Salz überführt, und/oder eine erhältliche freie Verbindung in ein Salz überführt und/oder ein erhältliches Gemisch von Isomeren in die einzelnen Isomere auftrennt.

2. Verfahren gemäss Anspruch 1 zur Herstellung von Verbindungen der Formel I, worin $R_1$ $C_1$-$C_7$-Alkyl, $R_2$ Wasserstoff, Sulfo, $C_1$-$C_7$-Alkanoyl oder $C_1$-$C_7$-Alkansulfonyl und $R_3$ Wasserstoff oder $C_1$-$C_7$-Alkyl bedeuten, und von Salzen dieser Verbindungen.

3. Verfahren gemäss Anspruch 1 zur Herstellung von Verbindungen der Formel I, worin $R_1$ $C_1$-$C_{12}$-Alkyl, $C_2$-$C_7$-Alkenyl, $C_2$-$C_7$-Alkinyl, $C_3$-$C_{10}$-Cycloalkyl oder $C_3$-$C_6$-Cycloalkyl-$C_1$-$C_4$-alkyl, $R_2$ Wasserstoff oder $C_1$-$C_7$-Alkyl und $R_3$ Wasserstoff oder $C_1$-$C_7$-Alkyl bedeuten, und von Salzen dieser Verbindugen.

4. Verfahren gemäss Anspruch 1 zur Herstellung von Verbindungen der Formel I, worin $R_1$ $C_1$-$C_7$-Alkyl und $R_2$ und $R_3$ Wasserstoff bedeuten, sowie von Salzen dieser Verbindungen.

5. Verfahren gemäss Anspruch 1 zur Herstellung von Verbindungen der Formel I, worin $R_1$ $C_1$-$C_7$-Alkyl, $C_2$-$C_4$-Alkenyl, $C_2$-$C_4$-Alkinyl oder $C_3$-$C_6$-Cycloalkyl-$C_1$-$C_4$-alkyl bedeutet, die Gruppe $-N(R_2)(R_3)$ sich in 4-Stellung des Phenylrests befindet und $R_2$ und $R_3$ Wasserstoff bedeuten, sowie von pharmazeutisch verwendbaren Salzen dieser Verbindungen.

6. Verfahren gemäss Anspruch 1 zur Herstellung von Verbindungen der Formel I, worin $R_1$ $C_1$-$C_7$-Alkyl bedeutet, die Gruppe $-N(R_2)(R_3)$ sich in 4-Stellung des Phenylrests befindet und $R_2$ und $R_3$ Wasserstoff bedeuten, sowie von pharmazeutisch verwendbaren Salzen dieser Verbindungen.

7. Verfahren gemäss Anspruch 1 zur Herstellung von 1-(4-Aminophenyl)-3-methyl-3-azabicyclo[3.1.0]hexan-2,4-dion.

8. Verfahren gemäss Anspruch 1 zur Herstellung von 1-(4-Aminophenyl)-3-n-propyl-3-azabicyclo[3.1.0]hexan-2,4-dion.

9. Verfahren gemäss Anspruch 1 zur Herstellung von 1-(4-Aminophenyl)-3-isobutyl-3-azabicyclo[3.1.0]hexan-2,4-dion.

10. Verfahren gemäss Anspruch 1 zur Herstellung von 1-(4-Aminophenyl)-3-n-butyl-3-azabicyclo[3.1.0]hexan-2,4-dion.

11. Verfahren gemäss Anspruch 1 zur Herstellung von 1-(4-Aminophenyl)-3-n-pentyl-3-azabicyclo[3.1.0]hexan-2,4-dion.

12. Verfahren gemäss Anspruch 1 zur Herstellung von 1-(4-Aminophenyl)-3-neopentyl-3-azabicyclo[3.1.0]hexan-2,4-dion.

13. Verfahren gemäss Anspruch 1 zur Herstellung von 1-(4-Aminophenyl)-3-n-heptyl-3-azabicyclo[3.1.0)hexan-2,4-dion.

14. Verfahren gemäss Anspruch 1 zur Herstellung von 1-(4-Aminophenyl)-3-cyclohexylmethyl-3-azabicyclo[3.1.0]hexan-2,4-dion.

15. Verfahren zur Herstellung von Verbindungen der Formel

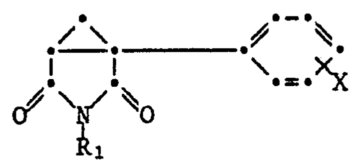

(II),

worin $R_1$ $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl und X Nitroso, Hydroxyamino, Carbamoyl, Azidocarbonyl oder geschütztes Amino bedeuten, und von Salzen dieser Verbindungen, dadurch gekennzeichnet, dass man eine Verbindung der Formel

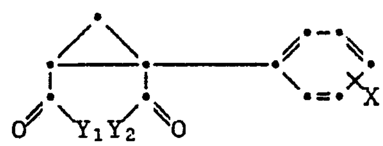

(VI),

worin $Y_1$ und $Y_2$ Abgangsgruppen und X' Nitroso, Hydroxyamino, Carboxyl oder geschütztes Amino darstellen, oder ein Salz davon durch Umsetzung mit einer die Gruppe -NH-$R_1$ liefernden Verbindung zyklisiert und in einer erhältlichen Verbindung die Carboxylgruppe X' in Carbamoyl oder Azidocarbonyl überführt und/oder gewünschtenfalls eine erfindungsgemäss erhältliche Verbindung der Formel II in eine andere definitionsgemässe Verbindung der Formel II umwandelt und/oder ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

16. Verfahren zur Herstellung von Verbindungen der Formel

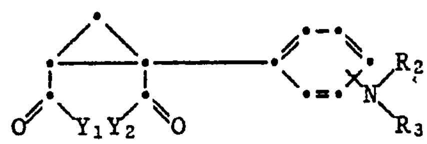

(III),

worin $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten und $Y_1$ und $Y_2$ Abgangsgruppen darstellen, und von Salzen dieser Verbindungen mit salzbildenden Gruppen, dadurch gekennzeichnet, dass man eine Verbindung der Formel

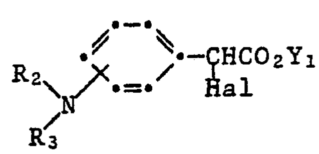

(VII),

worin $R_2$, $R_3$ und $Y_1$ die unter Formel III genannte Bedeutung haben und Hal Chlor oder Brom bedeutet, mit einem Acrylsäurederivat der Formel

$HC{=}CHCO_2Y_2$ (VIII),

worin $Y_2$ die unter Formel III genannte Bedeutung hat, umsetzt und gewünschtenfalls ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

17. Verfahren zur Herstellung von pharmazeutischen Präparaten enthaltend Verbindungen der Formel I, worin $R_1$ Wasserstoff, $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, oder Salze davon, dadurch gekennzeichnet, dass man Verbindungen der Formel I und Salze davon nach dem Verfahren gemäss Anspruch 1 herstellt und diese mit einem pharmazeutischen Trägermaterial mischt.

18. Verfahren gemäss Anspruch 17 zur Herstellung von pharmazeutischen Präparaten enthaltend 1-(4-Aminophenyl)-3-azabicyclo[3.1.0]hexan-2,4-dion.

**Patentansprüche** (für die Vertragsstaaten BE und DE)

1. Verbindungen der Formel

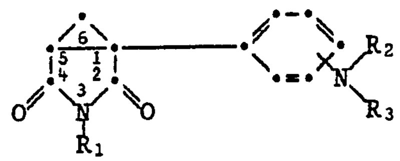

(I),

worin $R_1$ $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, und Salze dieser Verbindungen.

2. Verbindungen gemäss Anspruch 1 der Formel I, worin $R_1$ $C_1$-$C_7$-Alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, und Salze dieser Verbindungen.

3. Verbindungen gemäss Anspruch 1 der Formel I, worin $R_1$ $C_1$-$C_{12}$-Alkyl, $C_2$-$C_7$-Alkenyl, $C_2$-$C_7$-Alkinyl, $C_3$-$C_{10}$-Cycloalkyl oder $C_3$-$C_6$-Cycloalkyl-$C_1$-$C_4$-alkyl, $R_2$ Wasserstoff oder $C_1$-$C_7$-Alkyl und $R_3$ Wasserstoff oder $C_1$-$C_7$-Alkyl bedeuten, sowie Salze davon.

4. Verbindungen gemäss Anspruch 2 der Formel I, worin $R_1$ $C_1$-$C_7$-Alkyl und $R_2$ und $R_3$ Wasserstoff bedeuten, sowie Salze davon.

5. Verbindungen gemäss Anspruch 1 der Formel I, worin $R_1$ $C_1$-$C_7$-Alkyl, $C_2$-$C_4$-Alkenyl, $C_2$-$C_4$-Alkinyl oder $C_3$-$C_6$-Cycloalkyl-$C_1$-$C_4$-alkyl bedeutet, die Gruppe -N($R_2$)($R_3$) sich in 4-Stellung des Phenylrests befindet und $R_2$ und $R_3$ Wasserstoff bedeuten, sowie pharmazeutisch verwendbare Salze davon.

6. Verbindungen gemäss Anspruch 2 der Formel I, worin $R_1$ $C_1$-$C_7$-Alkyl bedeutet die Gruppe -N($R_2$)($R_3$) sich in 4-Stellung des Phenylrests befindet und $R_2$ und $R_3$ Wasserstoff bedeuten, sowie pharmazeutisch verwendbare Salze davon.

7. 1-(4-Aminophenyl)-3-methyl-3-azabicyclo[3.1.0]hexan-2,4-dion gemäss Anspruch 1.

8. 1-(4-Aminophenyl)-3-n-propyl-3-azabicyclo[3.1.0]hexan-2,4-dion gemäss Anspruch 1.

9. 1-(4-Aminophenyl)-3-isobutyl-3-azabicyclo[3.1.0)hexan-2,4-dion gemäss Anspruch 1.

10. 1-(4-Aminophenyl)-3-n-butyl-3-azabicyclo[3.1.0]hexan-2,4-dion gemäss Anspruch 1.

11. 1-(4-Aminophenyl)-3-n-pentyl-3-azabicyclo[3.1.0]hexan-2,4-dion gemäss Anspruch 1.

12. 1-(4-Aminophenyl)-3-neopentyl-3-azabicyclo[3.1.0]hexan-2,4-dion gemäss Anspruch 1.

13. 1-(4-Aminophenyl)-3-n-heptyl-3-azabicyclo[3.1.0]hexan-2,4-dion gemäss Anspruch 1.

14. 1-(4-Aminophenyl)-3-cyclohexylmethyl-3-azabicyclo[3.1.0]hexan-2,4-dion gemäss Anspruch 1.

15. Pharmazeutische Präparate enthaltend Verbindungen der Formel I, worin $R_1$ Wasserstoff, $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl, $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten, oder Salze davon zusammen mit einem pharmazeutisch verwendbaren Trägerstoff.

16. Pharmazeutische Präparate gemäss Anspruch 15 enthaltend 1-(4-Aminophenyl)-3-azabicyclo[3.1.0]hexan-2,4-dion.

17. Verbindungen der Formel I, worin $R_1$, $R_2$ und $R_3$ die in Anspruch 15 genannten Bedeutungen haben, oder Salze davon zur Anwendung in einem Verfahren zur therapeutischen Behandlung des menschlichen oder tierischen Körpers.

18. Verwendung von Verbindungen der Formel I, worin $R_1$, $R_2$ und $R_3$ die in Anspruch 15 genannten Bedeutungen haben, oder von Salzen davon zur Herstellung eines carcinostatischen Heilmittels.

19. Verfahren zur Herstellung von Verbindungen der Formel I gemäss Anspruch 1, und von Salzen dieser Verbindungen, dadurch gekennzeichnet, dass man

a) in einer Verbindung der Formel

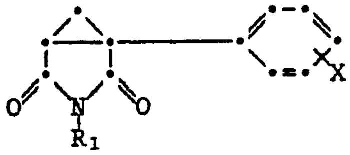

(II),

worin $R_1$ die unter Formel I genannte Bedeutung hat und X eine in die -N($R_2$)($R_3$)-Gruppe überführbare Gruppe darstellt, oder in einem Salz davon X in die -N($R_2$)($R_3$)-Gruppe überführt, oder

b) eine Verbindung der Formel

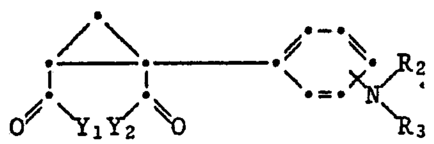

(III),

worin $R_2$ und $R_3$ die unter Formel I genannten Bedeutungen haben, $Y_1$ und $Y_2$ Abgangsgruppen darstellen, oder ein Salz davon durch Umsetzung mit einer die Gruppe

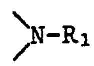

liefernden Verbindung zyklisiert,

oder

c) in einer Verbindung der Formel

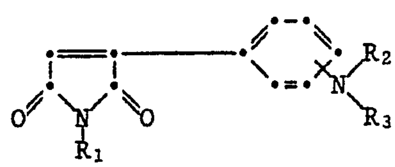

(IV),

worin $R_1$, $R_2$ und $R_3$ die unter Formel I genannte Bedeutung haben, oder in einem Salz davon $-CH_2-$ (Methylen) an die Doppelbindung des Maleimids anlagert, und

gewünschtenfalls eine erfindungsgemäss erhältliche Verbindung der Formel I in eine andere definitionsgemässe Verbindung der Formel umwandelt und/oder ein erhältliches Salz in die freie Verbindung oder in ein anderes Salz überführt, und/oder eine erhältliche freie Verbindung in ein Salz überführt und/oder ein erhältliches Gemisch von Isomeren in die einzelnen Isomere auftrennt.

20. Verbindungen der Formel

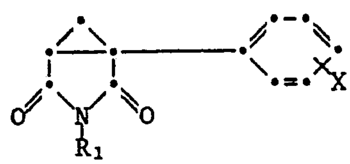

(II),

worin $R_1$ $C_1$-$C_{12}$-Alkyl, $C_2$-$C_{12}$-Alkenyl, $C_2$-$C_7$-Alkinyl, $C_3$-$C_{10}$-Cycloalkyl, $C_3$-$C_{10}$-Cycloalkenyl, $C_3$-$C_6$-Cycloalkyl-$C_1$-$C_4$-alkyl, $C_3$-$C_6$-Cycloalkyl-$C_2$-$C_4$-alkenyl oder $C_3$-$C_6$-Cycloalkenyl-$C_1$-$C_4$-alkyl und X Nitroso, Hydroxyamino, Carbamoyl, Azidocarbonyl oder geschütztes Amino bedeuten, und Salze von diesen Verbindungen.

21. Verfahren zur Herstellung von Verbindungen der Formel II gemäss Anspruch 20, und von Salzen dieser Verbindungen, dadurch gekennzeichnet, dass man eine Verbindung der Formel

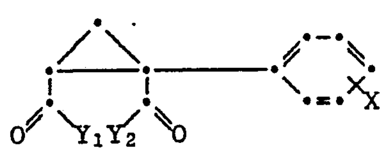

(VI),

worin $Y_1$ und $Y_2$ Abgangsgruppen und X' Nitroso, Hydroxyamino, Carboxyl oder geschütztes Amino darstellen, oder ein Salz davon durch Umsetzung mit einer die Gruppe $-NH-R_1$ liefernden Verbindung zyklisiert und in einer erhältlichen Verbindung die Carboxylgruppe X' in Carbamoyl oder Azidocarbonyl überführt und/oder gewünschtenfalls eine erfindungsgemäss erhältliche Verbindung der Formel II in eine andere definitionsgemässe Verbindung der Formel II umwandelt und/oder ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

22. Verbindungen der Formel

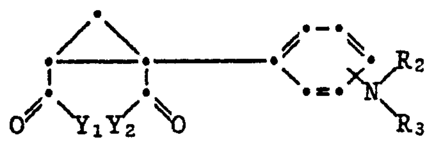

(III),

worin $R_2$ Wasserstoff, $C_1$-$C_7$-Alkyl, Sulfo, $C_1$-$C_7$-Alkanoyl oder $C_1$-$C_7$-Alkansulfonyl und $R_3$ Wasserstoff oder $C_1$-$C_7$-Alkyl bedeuten und $Y_1$ und $Y_2$ Abgangsgruppen darstellen, und Salze von diesen Verbindungen mit salzbildenden Gruppen.

23. Verfahren zur Herstellung von Verbindungen der Formel III gemäss Anspruch 22, und von Salzen dieser Verbindungen, dadurch gekennzeichnet, dass man eine Verbindung der Formel

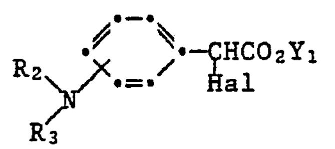

(VII),

worin $R_2$, $R_3$ und $Y_1$ die in Anspruch 22 genannten Bedeutungen haben und Hal Chlor oder Brom bedeutet, mit einem Acrylsäurederivat der Formel

$HC{=}CHCO_2Y_2$ (VIII),

worin $Y_2$ die in Anspruch 22 genannte Bedeutung hat, umsetzt und gewünschtenfalls ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

24. Verfahren zur Herstellung von pharmazeutischen Präparaten enthaltend Verbindungen der Formel I, worin $R_1$, $R_2$ und $R_3$ die in Anspruch 15 genannten Bedeutungen haben, oder Salze davon, dadurch gekennzeichnet, dass man Verbindungen der Formel I und Salze davon nach dem Verfahren gemäss Anspruch 19 herstellt und diese mit einem pharmazeutischen Trägermaterial mischt.

**Claims** for the Contracting States: FR, IT, LU, NL, SE, GB, CH/LI

1. Compounds of the formula

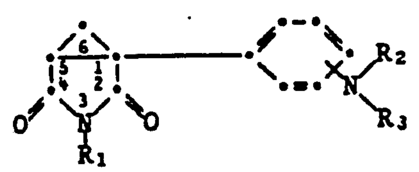

(I),

wherein $R_1$ is hydrogen, $R_2$ is hydrogen, sulfo, $C_1$-$C_7$-alkanoyl or $C_1$-$C_7$-alkanesulfonyl and $R_3$ is hydrogen, or $R_1$ is $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl, $C_3$-$C_6$-cycloalkyl-$C_2$-$C_4$-alkenyl or $C_3$-$C_6$-cycloalkenyl-$C_1$-$C_4$-alkyl, $R_2$ is hydrogen, $C_1$-$C_7$-alkyl, sulfo, $C_1$-$C_7$-alkanoyl or $C_1$-$C_7$-alkanesulfonyl and $R_3$ is hydrogen or $C_1$-$C_7$-alkyl, and salts thereof.

2. Compounds according to claim 1 of the formula I, wherein $R_1$ is hydrogen or $C_1$-$C_7$-alkyl, $R_2$ is hydrogen, $C_1$-$C_7$-alkyl, sulfo, $C_1$-$C_7$-alkanoyl or $C_1$-$C_7$-alkanesulfonyl, and $R_3$ is hydrogen or $C_1$-$C_7$-alkyl, and salts thereof.

3. Compounds according to claim 1 of the formula I, wherein $R_1$, $R_2$ and $R_3$ are hydrogen or wherein $R_1$ is $C_1$-$C_{12}$-alkyl, $C_2$-$C_7$-alkenyl, $C_2$-$C_7$-alkynyl, $C_3$-$C_{10}$-cycloalkyl or $C_3$-$C_6$-cycloalkyl-$C_1$-$C_4$-alkyl, $R_2$ is hydrogen or $C_1$-$C_7$-alkyl, and $R_3$ is hydrogen or $C_1$-$C_7$-alkyl, and salts thereof.

4. Compounds according to claim 2 of the formula I, wherein $R_1$ is hydrogen or $C_1$-$C_7$-alkyl and $R_2$ and $R_3$ are hydrogen, and salts thereof.

5. Compounds according to claim 1 of the formula I, wherein $R_1$ is hydrogen, $C_1$-$C_7$-alkyl, $C_2$-$C_4$-alkenyl, $C_2$-$C_4$-alkynyl or $C_3$-$C_6$-cycloalkyl-$C_1$-$C_4$-alkyl, the group -$N(R_2)(R_3)$ is in the 4-position of the phenyl radical and $R_2$ and $R_3$ are hydrogen, and pharmaceutically acceptable salts thereof.

6. Compounds according to claim 2 of the formula I, wherein $R_1$ is hydrogen or $C_1$-$C_7$-alkyl, the group -$N(R_2)(R_3)$ is in the 4-position of the phenyl radical and $R_2$ and $R_3$ are hydrogen, and pharmaceutically acceptable salts thereof.

7. 1-(4-Aminophenyl)-3-methyl-3-azabicyclo[3.1.0]hexane-2,4-dione, according to claim 1.

8. 1-(4-Aminophenyl)-3-n-propyl-3-azabicyclo[3.1.0]hexane-2,4-dione, according to claim 1.

9. 1-(4-Aminophenyl)-3-isobutyl-3-azabicyclo[3.1.0]hexane-2,4-dione, according to claim 1.

10. 1-(4-Aminophenyl)-3-n-butyl-3-azabicyclo[3.1.0]hexane-2,4-dione, according to claim 1.

11. 1-(4-Aminophenyl)-3-n-pentyl-3-azabicyclo[3.1.0]hexane-2,4-dione, according to claim 1.

12. 1-(4-Aminophenyl)-3-neopentyl-3-azabicyclo[3.1.0]hexane-2,4-dione, according to claim 1.

13. 1-(4-Aminophenyl)-3-n-heptyl-3-azabicyclo[3.1.0]hexane-2,4-dione, according to claim 1.

14. 1-(4-Aminophenyl)-3-cyclohexylmethyl-3-azabicyclo[3.1.0]hexane-2,4-dione, according to claim 1.

15. Pharmaceutical compositions which contain compounds of the formula I, wherein $R_1$ is hydrogen, $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl, $C_3$-$C_6$-cycloalkyl-$C_2$-$C_4$-alkenyl or $C_3$-$C_6$-cycloalkenyl-$C_1$-$C_4$-alkyl, $R_2$ is hydrogen, $C_1$-$C_7$-alkyl, sulfo, $C_1$-$C_7$-alkanoyl or $C_1$-$C_7$-alkanesulfonyl, and $R_3$ is hydrogen or $C_1$-$C_7$-alkyl, or salts thereof, together with a pharmaceutically acceptable carrier.

16. Pharmaceutical compositions according to claim 15 which contain 1-(4-aminophenyl)-3-azabicyclo[3.1.0]-hexane-2,4-dione.

17. Compounds of the formula I, wherein $R_1$, $R_2$ and $R_3$ are as defined in claim 15, or salts thereof, for use in a therapeutic method of treating the human or animal body.

18. The use of compounds of the formula I, wherein $R_1$, $R_2$ and $R_3$ are as defined in claim 15, or salts thereof, for the preparation of a carcinostatic medicament.

19. A process for the preparation of compounds of the formula I according to claim 1, and of salts thereof, which process comprises

a) converting X into the -N($R_2$)($R_3$) group in a compound of the formula

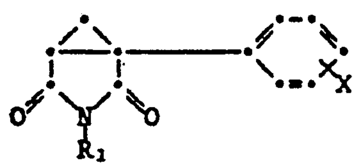

(II)

wherein $R_1$ is as defined for formula I and X is a group which can be converted into the -N($R_2$)($R_3$) group, or a salt thereof, or

b) cyclising a compound of the formula

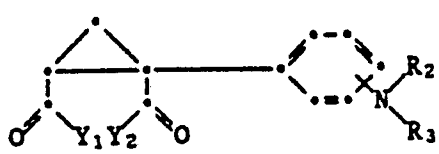

(III)

wherein $R_2$ and $R_3$ are as defined for formula I and $Y_1$ and $Y_2$ are leaving groups, or a salt thereof, by reaction with a compound which introduces the group

$$>N-R_1,$$

or

c) by addition of -$CH_2$- (methylene) to the double bond of the maleimide in a compound of the formula

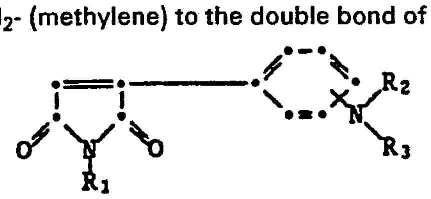

(IV)

wherein $R_1$, $R_2$ and $R_3$ are as defined for formula I, or a salt thereof,

and, if desired, converting a compound of the formula I obtained in accordance with the invention into another compound of the formula I according to the definition, and/or converting a resultant salt into the free compound or into another salt, and/or converting a resultant free compound into a salt, and/or separating a resultant mixture of isomers into the individual isomers.

20. Compounds of the formula

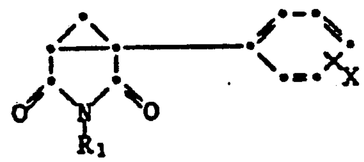

(II)

wherein $R_1$ is hydrogen $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl, $C_3$-$C_6$-cycloalkyl-$C_2$-$C_4$-alkenyl or $C_3$-$C_6$-cycloalkenyl-$C_1$-$C_4$-alkyl and X is nitroso, hydroxylamino, carbamoyl, azidocarbonyl or protected amino, and salts thereof.

21. A process for the preparation of compounds of the formula II according to claim 20, and of salts thereof, which process comprises cyclising a compound of the formula

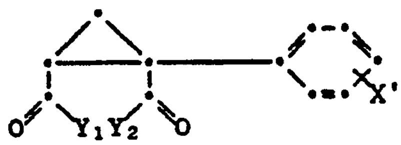

(VI)

wherein $Y_1$ and $Y_2$ are leaving groups and X' is nitroso, hydroxylamino, carboxyl or protected amino, or a salt thereof, by reaction with a compound which introduces the group -NH-$R_1$ and, in a resultant compound, converting the carboxyl group X' into carbamoyl or azidocarbonyl, and/or, if desired, converting a compound of the formula II obtained in accordance with the invention into another compound of the formula II according to the definition, and/or converting a resultant salt into the free compound or into another salt, and/or converting a resultant free compound into a salt, and/or separating a resultant mixture of isomers into the individual isomers.

22. Compounds of the formula

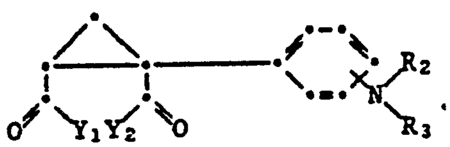

(III)

wherein $R_2$ is hydrogen $C_1$-$C_7$-alkyl, sulfo, $C_1$-$C_7$-alkanoyl or $C_1$-$C_7$-alkanesulfonyl, $R_3$ is hydrogen or $C_1$-$C_7$-alkyl, and $Y_1$ and $Y_2$ are leaving groups, and salts of such compounds containing salt-forming groups.

23. A process for the preparation of compounds of the formula III according to claim 22, and of salts thereof, which process comprises reacting a compound of the formula

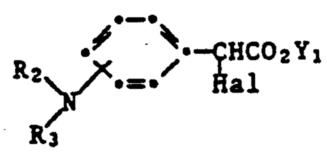

(VII),

wherein $R_2$, $R_3$ and $Y_1$ are as defined in claim 22 and Hal is chlorine or bromine, with an acrylic acid derivative of the formula

$HC=CHCO_2Y_2$ (VIII)

wherein $Y_2$ is as defined in claim 22, and, if desired, converting a resultant salt into the free compound or into another salt, and/or converting a resultant free compound into a salt, and/or separating a resultant mixture of isomers into the individual isomers.

24. A process for the preparation of pharmaceutical compositions which contain compounds of the formula I, wherein $R_1$, $R_2$ and $R_3$ are as defined in claim 15, or salts thereof, which process comprises preparing compounds of the formula I and salts thereof by the process as claimed in claim 19, and mixing them with a pharmaceutical carrier.

**Claims** for the Contracting State: AT

1. A process for the preparation of compounds of the formula

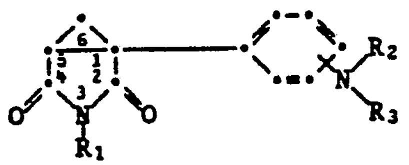

(I),

wherein $R_1$ is $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl, $C_3$-$C_6$-cycloalkyl-$C_2$-$C_4$-alkenyl or $C_3$-$C_6$-cycloalkenyl-$C_1$-$C_4$-alkyl, $R_2$ is hydrogen, $C_1$-$C_7$-alkyl, sulfo, $C_1$-$C_7$-alkanoyl or $C_1$-$C_7$-alkanesulfonyl and $R_3$ is hydrogen or $C_1$-$C_7$-alkyl, and salts thereof, which process comprises

a) converting X into the -N($R_2$)($R_3$) group in a compound of the formula

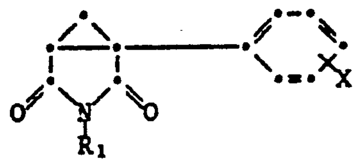

(II)

wherein $R_1$ is as defined for formula I and X is a group which can be converted into the -N($R_2$)($R_3$) group, or in a salt thereof, or

b) cyclising a compound of the formula

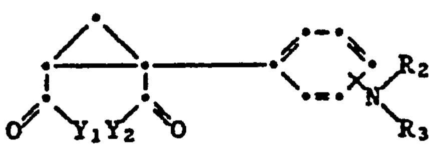

(III)

wherein $R_2$ and $R_3$ are as defined for formula I and $Y_1$ and $Y_2$ are leaving groups, or a salt thereof, by reaction with a compound which introduces the group

$>N-R_1$,

or

c) by addition of $-CH_2-$ (methylene) to the double bond of the maleimide in a compound of the formula

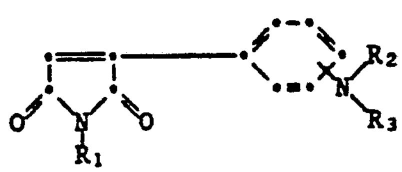

(IV)

wherein $R_1$, $R_2$ and $R_3$ are as defined for formula I, or a salt thereof,

and, if desired, converting a compound of the formula I obtained in accordance with the invention into another compound of the formula I according to the definition, and/or converting a resultant salt into the free compound or into another salt, and/or converting a resultant free compound into a salt, and/or separating a resultant mixture of isomers into the individual isomers.

2. A process according to claim 1 for the preparation of compounds of the formula I, wherein $R_1$ is $C_1$-$C_7$-alkyl, $R_2$ is hydrogen, sulfo, $C_1$-$C_7$-alkanoyl or $C_1$-$C_7$-alkanesulfonyl, and $R_3$ is hydrogen or $C_1$-$C_7$-alkyl, and salts thereof.

3. A process according to claim 1 for the preparation of compounds of the formula I, wherein $R_1$ is $C_1$-$C_{12}$-alkyl, $C_2$-$C_7$-alkenyl, $C_2$-$C_7$-alkynyl, $C_3$-$C_{10}$-cycloalkyl or $C_3$-$C_6$-cycloalkyl-$C_1$-$C_4$-alkyl, $R_2$ is hydrogen or $C_1$-$C_7$-alkyl, and $R_3$ is hydrogen or $C_1$-$C_7$-alkyl, and salts thereof.

4. A process according to claim 1 for the preparation of compounds of the formula I, wherein $R_1$ is $C_1$-$C_7$-alkyl

and $R_2$ and $R_3$ are hydrogen, and salts thereof.

5. A process according to claim 1 for the preparation of compounds of the formula I, wherein $R_1$ is $C_1$-$C_7$-alkyl, $C_2$-$C_4$-alkenyl, $C_2$-$C_4$-alkynyl or $C_3$-$C_6$-cycloalkyl-$C_1$-$C_4$-alkyl, the group -N($R_2$)($R_3$) is in the 4-position of the phenyl radical and $R_2$ and $R_3$ are hydrogen, and pharmaceutically acceptable salts thereof.

6. A process according to claim 1 for the preparation of compounds of the formula I, wherein $R_1$ is $C_1$-$C_7$-alkyl, the group -N($R_2$)($R_3$) is in the 4-position of the phenyl radical and $R_2$ and $R_3$ are hydrogen, and pharmaceutically acceptable salts thereof.

7. A process according to claim 1 for the preparation of 1-(4-aminophenyl)-3-methyl-3-azabicyclo[3.1.0]hexane-2,4-dione.

8. A process according to claim 1 for the preparation of 1-(4-aminophenyl)-3-n-propyl-3-azabicyclo[3.1.0]-hexane-2,4-dione.

9. A process according to claim 1 for the preparation of 1-(4-aminophenyl)-3-isobutyl-3-azabicyclo[3.1.0]-hexane-2,4-dione.

10. A process according to claim 1 for the preparation of 1-(4-aminophenyl)-3-n-butyl-3-azabicyclo[3.1.0]-hexane-2,4-dione.

11. A process according to claim 1 for the preparation of 1-(4-aminophenyl)-3-n-pentyl-3-azabicyclo[3.1.0]-hexane-2,4-dione.

12. A process according to claim 1 for the preparation of 1-(4-aminophenyl)-3-neopentyl-3-azabicyclo[3.1.0]-hexane-2,4-dione.

13. A process according to claim 1 for the preparation of 1-(4-aminophenyl)-3-n-heptyl-3-azabicyclo[3.1.0]-hexane-2,4-dione.

14. A process according to claim 1 for the preparation of 1-(4-aminophenyl)-3-cyclohexylmethyl-3-azabicyclo-[3.1.0]hexane-2,4-dione.

15. A process for the preparation of compounds of the formula

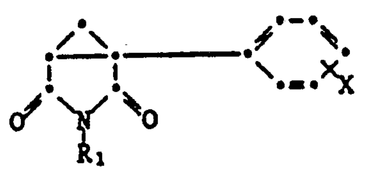

(II)

wherein $R_1$ is $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl, $C_3$-$C_6$-cycloalkyl-$C_2$-$C_4$-alkenyl or $C_3$-$C_6$-cycloalkenyl-$C_1$-$C_4$-alkyl and X is nitroso, hydroxylamino, carbamoyl, azidocarbonyl or protected amino, and salts thereof, which process comprises cyclising a compound of the formula

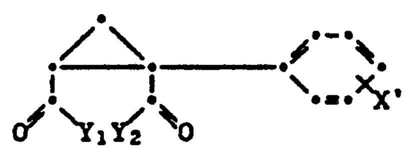

(VI)

wherein $Y_1$ and $Y_2$ are leaving groups and X' is nitroso, hydroxylamino, carboxyl or protected amino, or a salt thereof, by reaction with a compound which introduces the group -NH-$R_1$ and, in a resultant compound, converting the carboxyl group X' into carbamoyl or azidocarbonyl, and/or, if desired, converting a compound of the formula II obtained in accordance with the invention into another compound of the formula II according to the definition, and/or converting a resultant salt into the free compound or into another salt, and/or converting a resultant free compound into a salt, and/or separating a resultant mixture of isomers into the individual isomers.

16. A process for the preparation of compounds of the formula

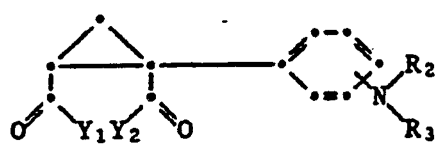

(III)

wherein $R_2$ is hydrogen, $C_1$-$C_7$-alkyl, sulfo, $C_1$-$C_7$-alkanoyl or $C_1$-$C_7$-alkanesulfonyl, $R_3$ is hydrogen or $C_1$-$C_7$-alkyl, and $Y_1$ and $Y_2$ are leaving groups, and salts of such compounds containing salt-forming groups, which process comprises reacting a compound of the formula

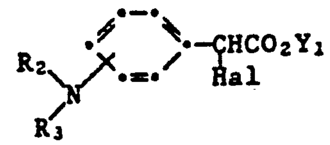

(VII),

wherein $R_2$, $R_3$ and $Y_1$ are as defined for formula III and Hal is chlorine or bromine, with an acrylic acid derivative of the formula

$HC=CHCO_2Y_2$ (VIII)

wherein $Y_2$ is as defined for formula III, and, if desired, converting a resultant salt into the free compound or into another salt, and/or converting a resultant free compound into a salt, and/or separating a resultant mixture of isomers into the individual isomers.

17. A process for the preparation of pharmaceutical compositions which contain compounds of the formula I, wherein $R_1$ is hydrogen, $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl; $C_3$-$C_6$-cycloalkyl-$C_2$-$C_4$-alkenyl or $C_3$-$C_6$-cycloalkenyl-$C_1$-$C_4$-alkyl, $R_2$ is hydrogen, $C_1$-$C_7$-alkyl, sulfo, $C_1$-$C_7$-alkanoyl or $C_1$-$C_7$-alkanesulfonyl and $R_3$ is hydrogen or $C_1$-$C_7$-alkyl, or salts thereof, which process comprises preparing compounds of the formula I and salts thereof by the process as claimed in claim 1, and mixing them with a pharmaceutical carrier.

18. A process according to claim 17 for the preparation of pharmaceutical compositions which contain 1-(4-aminophenyl)-3-azabicyclo[3.1.0]hexane-2,4-dione.

**Claims** for the Contracting States: BE and DE

1. Compounds of the formula

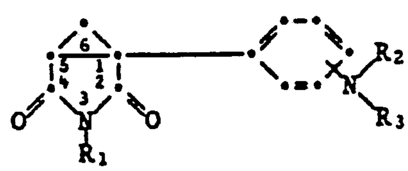

(I),

wherein $R_1$ is $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl, $C_3$-$C_6$-cycloalkyl-$C_2$-$C_4$-alkenyl or $C_3$-$C_6$-cycloalkenyl-$C_1$-$C_4$-alkyl, $R_2$ is hydrogen, $C_1$-$C_7$-alkyl, sulfo, $C_1$-$C_7$-alkanoyl or $C_1$-$C_7$-alkanesulfonyl and $R_3$ is hydrogen or $C_1$-$C_7$-alkyl, and salts thereof.

2. Compounds according to claim 1 of the formula I, wherein $R_1$ is $C_1$-$C_7$-alkyl, $R_2$ is hydrogen, $C_1$-$C_7$-alkyl, sulfo, $C_1$-$C_7$-alkanoyl or $C_1$-$C_7$-alkanesulfonyl, and $R_3$ is hydrogen or $C_1$-$C_7$-alkyl, and salts thereof.

3. Compounds according to claim 1 of the formula I, wherein $R_1$ is $C_1$-$C_{12}$-alkyl, $C_2$-$C_7$-alkenyl, $C_2$-$C_7$-alkynyl, $C_3$-$C_{10}$-cycloalkyl or $C_3$-$C_6$-cycloalkyl-$C_1$-$C_4$-alkyl, $R_2$ is hydrogen or $C_1$-$C_7$-alkyl, and $R_3$ is hydrogen or $C_1$-$C_7$-alkyl, and salts thereof.

4. Compounds according to claim 2 of the formula I, wherein $R_1$ is $C_1$-$C_7$-alkyl and $R_2$ and $R_3$ are hydrogen, and salts thereof.

5. Compounds according to claim 1 of the formula I, wherein $R_1$ is $C_1$-$C_7$-alkyl, $C_2$-$C_4$-alkenyl, $C_2$-$C_4$-alkynyl or $C_3$-$C_6$-cycloalkyl-$C_1$-$C_4$-alkyl, the group -N($R_2$)($R_3$) is in the 4-position of the phenyl radical and $R_2$ and $R_3$ are hydrogen, and pharmaceutically acceptable salts thereof.

6. Compounds according to claim 2 of the formula I, wherein $R_1$ is $C_1$-$C_7$-alkyl, the group -N($R_2$)($R_3$) is in the 4-position of the phenyl radical and $R_2$ and $R_3$ are hydrogen, and pharmaceutically acceptable salts thereof.

7. 1-(4-Aminophenyl)-3-methyl-3-azabicyclo[3.1.0]hexane-2,4-dione, according to claim 1.

8. 1-(4-Aminophenyl)-3-n-propyl-3-azabicyclo[3.1.0]hexane-2,4-dione, according to claim 1.

9. 1-(4-Aminophenyl)-3-isobutyl-3-azabicyclo[3.1.0]hexane-2,4-dione, according to claim 1.

10. 1-(4-Aminophenyl)-3-n-butyl-3-azabicyclo[3.1.0]hexane-2,4-dione, according to claim 1.

11. 1-(4-Aminophenyl)-3-n-pentyl-3-azabicyclo[3.1.0]hexane-2,4-dione, according to claim 1.

12. 1-(4-Aminophenyl)-3-neopentyl-3-azabicyclo[3.1.0]hexane-2,4-dione, according to claim 1.

13. 1-(4-Aminophenyl)-3-n-heptyl-3-azabicyclo[3.1.0]hexane-2,4-dione, according to claim 1.

14. 1-(4-Aminophenyl)-3-cyclohexylmethyl-3-azabicyclo[3.1.0]hexane-2,4-dione, according to claim 1.

15. Pharmaceutical compositions which contain compounds of the formula I, wherein $R_1$ is hydrogen, $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl, $C_3$-$C_6$-cycloalkyl-$C_2$-$C_4$-alkenyl or $C_3$-$C_6$-cycloalkenyl-$C_1$-$C_4$-alkyl, $R_2$ is hydrogen, $C_1$-$C_7$-alkyl, sulfo, $C_1$-$C_7$-alkanoyl or $C_1$-$C_7$-alkanesulfonyl, and $R_3$ is hydrogen or $C_1$-$C_7$-alkyl, or salts thereof, together with a pharmaceutically

acceptable carrier.

16. Pharmaceutical compositions according to claim 15 which contain 1-(4-aminophenyl)-3-azabicyclo[3.1.0]-hexane-2,4-dione.

17. Compounds of the formula I, wherein $R_1$, $R_2$ and $R_3$ are as defined in claim 15, or salts thereof, for use in a therapeutic method of treating the human or animal body.

18. The use of compounds of the formula I, wherein $R_1$, $R_2$ and $R_3$ are as defined in claim 15, or salts thereof, for the preparation of a carcinostatic medicament.

19. A process for the preparation of compounds of the formula I according to claim 1, and of salts thereof, which process comprises

a) converting X into the -N($R_2$)($R_3$) group in a compound of the formula

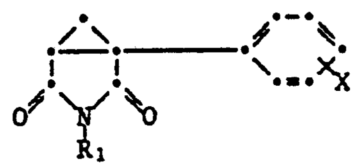

(II)

wherein $R_1$ is as defined for formula I and X is a group which can be converted into the -N($R_2$)($R_3$) group, or a salt thereof, or

b) cyclising a compound of the formula

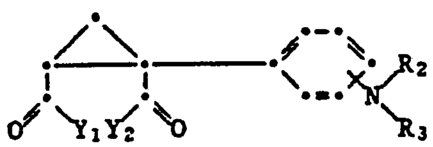

(III)

wherein $R_2$ and $R_3$ are as defined for formula I and $Y_1$ and $Y_2$ are leaving groups, or a salt thereof, by reaction with a compound which introduces the group

$>N-R_1$,

or

c) by addition of -$CH_2$- (methylene) to the double bond of the maleimide in a compound of the formula

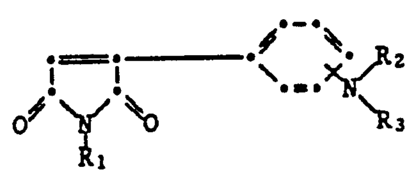

(IV)

wherein $R_1$, $R_2$ and $R_3$ are as defined for formula I, or a salt thereof,

and, if desired, converting a compound of the formula I obtained in accordance with the invention into another compound of the formula I according to the definition, and/or converting a resultant salt into the free compound or into another salt, and/or converting a resultant free compound into a salt, and/or separating a resultant mixture of isomers into the individual isomers.

20. Compounds of the formula

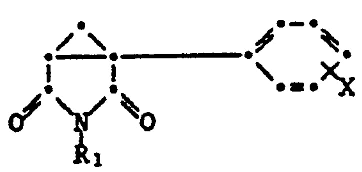

(II)

wherein $R_1$ is $C_1$-$C_{12}$-alkyl, $C_2$-$C_{12}$-alkenyl, $C_2$-$C_7$-alkynyl, $C_3$-$C_{10}$-cycloalkyl, $C_3$-$C_{10}$-cycloalkenyl, $C_3$-$C_6$-cycloalkyl-$C_1$-$C_4$-alkyl, $C_3$-$C_6$-cycloalkyl-$C_2$-$C_4$-alkenyl or $C_3$-$C_6$-cycloalkenyl-$C_1$-$C_4$-alkyl and X is nitroso, hydroxylamino, carbamoyl, azidocarbonyl or protected amino, and salts thereof.

21. A process for the preparation of compounds of the formula II according to claim 20, and of salts thereof,

which process comprises cyclising a compound of the formula

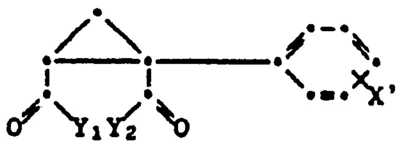

(VI)

wherein $Y_1$ and $Y_2$ are leaving groups and X' is nitroso, hydroxylamino, carboxyl or protected amino, or a salt thereof, by reaction with a compound which introduces the group $-NH-R_1$ and, in a resultant compound, converting the carboxyl group X' into carbamoyl or azidocarbonyl, and/or, if desired, converting a compound of the formula II obtained in accordance with the invention into another compound of the formula II according to the definition, and/or converting a resultant salt into the free compound or into another salt, and/or converting a resultant free compound into a salt, and/or separating a resultant mixture of isomers into the individual isomers.

22. Compounds of the formula

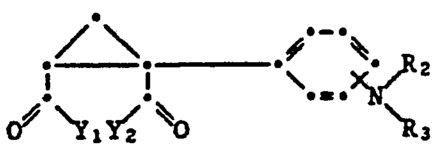

(III)

wherein $R_2$ is hydrogen, $C_1$-$C_7$-alkyl, sulfo, $C_1$-$C_7$-alkanoyl or $C_1$-$C_7$-alkanesulfonyl, $R_3$ is hydrogen or $C_1$-$C_7$-alkyl, and $Y_1$ and $Y_2$ are leaving groups, and salts of such compounds containing salt-forming groups.

23. A process for the preparation of compounds of the formula III according to claim 22, and of salts thereof, which process comprises reacting a compound of the formula

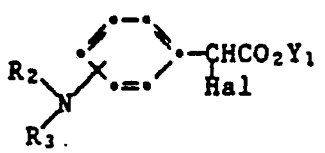

(VII),

wherein $R_2$, $R_3$ and $Y_1$ are as defined in claim 22 and Hal is chlorine or bromine, with an acrylic acid derivative of the formula

$HC=CHCO_2Y_2$ (VIII)

wherein $Y_2$ is as defined in claim 22, and, if desired, converting a resultant salt into the free compound or into another salt, and/or converting a resultant free compound into a salt, and/or separating a resultant mixture of isomers into the individual isomers.

24. A process for the preparation of pharmaceutical compositions which contain compounds of the formula I, wherein $R_1$, $R_2$ and $R_3$ are as defined in claim 15, or salts thereof, which process comprises preparing compounds of the formula I and salts thereof by the process as claimed in claim 19, and mixing them with a pharmaceutical carrier.

**Revendications** pour les Etats contractants: FR, IT, LU, NL, SE, GB, CH/LI

1. Composés répondant à la formule

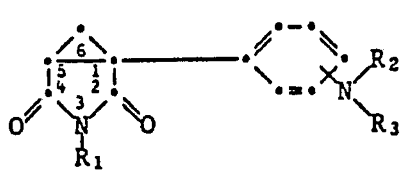

(I),

dans laquelle $R_1$ désigne un atome d'hydrogène, $R_2$ un atome d'hydrogène un groupe sulfo, alcanoyle en $C_1$-$C_7$ ou alcansulfonyle en $C_1$-$C_7$ et $R_3$ un atome d'hydrogène, ou dans laquelle $R_1$ désigne un groupe alkyle en $C_1$-

$C_{12}$, alcényle en $C_2$-$C_{12}$, alcinyle en $C_2$-$C_7$, cycloalkyle en $C_3$-$C_{10}$, cylcloalcényle en $C_3$-$C_{10}$, (cycloalkyle en $C_3$-$C_6$)(alkyle en $C_1$-$C_4$), (cycloalkyle en $C_3$-$C_6$)(alcény'e en $C_2$-$C_4$), ou (cycloalcényle en $C_3$-$C_6$)(alkyle en $C_1$-$C_4$), $R_2$ désigne un atome d'hydrogène, un groupe alkyle en $C_1$-$C_7$, sulfo, alcanoyle en $C_1$-$C_7$ ou alcansulfonyle en $C_1$-$C_7$ et $R_3$ désigne un atome d'hydrogène ou un groupe alkyle en $C_1$-$C_7$, et les sels de ces composés.

2. Composés selon la revendication 1 répondant à la formule I, dans laquelle $R_1$ désigne un atome d'hydrogène ou un groupe alkyle en $C_1$-$C_7$, $R_2$ un atome d'hydrogène, un groupe alkyle en $C_1$-$C_7$, sulfo, alcanoyle en $C_1$-$C_7$ ou alcansulfonyle en $C_1$-$C_7$ et $R_3$ un atome d'hydrogène ou un groupe alkyle en $C_1$-$C_7$, et les sels de ces composés.

3. Composés selon la revendication 1, répondant à la formule I, dans laquelle $R_1$, $R_2$ et $R_3$ désignent un atome d'hydrogène ou dans laquelle $R_1$ désigne un groupe alkyle en $C_1$-$C_{12}$, alcényle en $C_2$-$C_7$, alcinyle en $C_2$-$C_7$, cycloalkyle en $C_3$-$C_{10}$ ou (cycloalkyle en $C_3$-$C_6$)(alkyle en $C_1$-$C_4$), $R_2$ désigne un atome d'hydrogène ou un groupe alkyle en $C_1$-$C_7$ et $R_3$ un atome d'hydrogène ou un groupe alkyle en $C_1$-$C_7$, ainsi que leurs sels.

4. Composés selon la revendication 2, répondant à la formule I, dans laquelle $R_1$ désigne un atome d'hydrogène ou un groupe alkyle en $C_1$-$C_7$ et $R_2$ et $R_3$ désignent un atome d'hydrogène ainsi que leurs sels.

5. Composés selon la revendication 1, répondant à la formule I, dans laquelle $R_1$ désigne un atome d'hydrogène, un groupe alkyle en $C_1$-$C_7$, alcényle en $C_2$-$C_4$, alcinyle en $C_2$-$C_4$ ou (cycloalkyle en $C_3$-$C_6$)(alkyle en $C_1$-$C_4$), le groupe -$N(R_2)(R_3)$ se trouve à la position 4 du radical phényle et $R_2$ et $R_3$ désignent un atome d'hydrogène, ainsi que leurs sels pharmaceutiquement acceptables.

6. Composés selon la revendication 2, répondant à la fomule I, dans laquelle $R_1$ désigne un atome d'hydrogène ou un radical alkyle en $C_1$-$C_7$, le groupe -$N(R_2)(R_3)$ se trouve en position 4 du radical phényle et $R_2$ et $R_3$ désignent un atome d'hydrogène, ainsi que leurs sels pharmaceutiquement acceptables.

7. 1-(4-Aminophényl)-3-méthyl-3-azabicyclo[3.1.0]hexane-2,4-dione selon la revendication 1.

8. 1-(4-Aminophényl)-3-n-propyl-3-azabicyclo[3.1.0]hexane-2,4-dione selon la revendication 1.

9. 1-(4-Aminophényl)-3-isobutyl-3-azabicyclo[3.1.0]hexane-2,4-dione selon la revendication 1.

10. 1-(4-Aminophényl)-3-n-butyl-3-azabicyclo[3.1.0]hexane-2,4-dione selon la revendication 1.

11. 1-(4-Aminophényl)-3-n-pentyl-3-azabicyclo[3.1.0]hexane-2,4-dione selon la revendication 1.

12. 1-(4-Aminophényl)-3 -néopentyl-3-azabicyclo[3.1.0]hexane-2,4-dione selon la revendication 1.

13. 1-(4-Aminophényl)-3-n-heptyl-3-azabicyclo[3.1.0]hexane-2,4-dione selon la revendication 1.

14. 1-(4-Aminophényl)-3-cyclohexylméthyl-3-azabicyclo[3.1.0]hexane-2,4-dione selon la revendication 1.

15. Préparations pharmaceutiques contenant des composés répondant à la formule I, dans laquelle $R_1$ désigne un atome d'hydrogène, un groupe alkyle en $C_1$-$C_{12}$, alcényle en $C_2$-$C_{12}$, alcinyle en $C_2$-$C_7$, cycloalkyle en $C_3$-$C_{10}$, cylcloalcényle en $C_3$-$C_{10}$, (cycloalkyle en $C_3$-$C_6$)(alkyl en $C_1$-$C_4$), (cycloalkyle en $C_3$-$C_6$)(alcényle en $C_2$-$C_4$), ou (cycloalcényle en $C_3$-$C_6$)(alkyle en $C_1$-$C_4$), $R_2$ désigne un atome d'hydrogène, un groupe alkyle en $C_1$-$C_7$, sulfo, alcanoyle en $C_1$-$C_7$ ou alcansulfonyle en $C_1$-$C_7$ et $R_3$ désigne un atome d'hydrogène ou un groupe alkyle en $C_1$-$C_7$, ou leurs sels avec un support pharmaceutiquement acceptables.

16. Préparations pharmaceutiques selon la revendication 15, contenant de la 1-(4-aminophényl)-3-azabicyclo[3.1.0]hexane-2,4-dione.

17. Composés répondant à la formule I, dans laquelle $R_1$, $R_2$ et $R_3$ ont les significations indiquées dans la revendication 15 ou leurs sels pour l'utilisation dans un procédé de traitement thérapeutique de l'organisme humain ou animal.

18. Utilisation de composés répondant à la formule I, dans laquelle $R_1$, $R_2$ et $R_3$ ont les significations indiquées dans la revendication 15, ou de leurs sels, pour la préparation d'un médicament carcinostatique.

19. Procédé de préparation de composés répondant à la formule I, selon la revendication 1, et de sels de ces composés caractérisé en ce que

a) dans un composé répondant à la formule

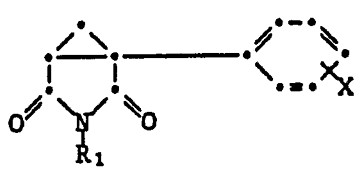

(II),

dans laquelle $R_1$ a la signification indiquée à propos de la formule I et X représente un groupe transformable en le groupe -$N(R_2)(R_3)$-, ou dans un sel de celui-ci on transforme X en le groupe -$N(R_2)(R_3)$-, ou

b) on cyclise un composé répondant à la formule

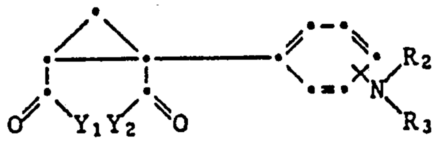

(III),

dans laquelle $R_2$ et $R_3$ ont la signification indiquée à propos de la formule I, $Y_1$ et $Y_2$ représentent des groupes partants ou un sel de celui-ci par réaction avec un composé fournissant le groupe

$$N-R_1$$

ou

c) dans un composé répondant à la formule

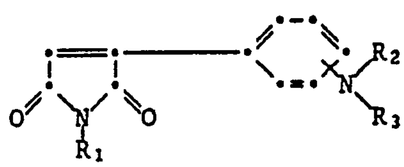

(IV),

dans laquelle $R_1$, $R_2$ et $R_3$ ont les significations indiquées à propos de la formule I, ou dans un sel de celui-ci on fixe par addition le groupe $-CH_2-$(méthylène) sur la double liaison du maléimide, et si on le désire, on transforme un composé répondant à la formule I pouvant être obtenu conformément à l'invention en un autre composé répondant à la formule I conforme à la définition et/ou on transforme un sel pouvant être obtenu en le composé libre ou en un autre sel, et/ou on transforme un composé libre pouvant être obtenu en un sel et/ou on sépare un mélange d'isomères pouvant être obtenu en les divers isomères.

20. Composés répondant à la formule

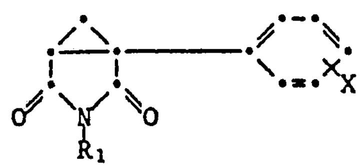

(II),

dans laquelle $R_1$ désigne un atome d'hydrogène, un groupe alkyle en $C_1$-$C_{12}$, alcényle en $C_2$-$C_{12}$, alcinyle en $C_2$-$C_7$, cycloalkyle en $C_3$-$C_{10}$, cycloalcényle en $C_3$-$C_{10}$, (cycloalkyle en $C_3$-$C_6$)(alkyle en $C_1$-$C_4$), (cycloalkyle en $C_3$-$C_6$)(alcényle en $C_2$-$C_4$), ou (cycloalcényle en $C_3$-$C_6$)(alkyle en $C_1$-$C_4$) et X désigne un groupe nitroso, hydroxyamino, carbamoyle, azidocarbonyle ou amino protégé, et les sels de ces composés.

21. Procédé de préparation de composés répondant à la formule II selon la revendication 20, et de sels de ces composés,, caractérisé en ce qu'on cyclise un composé répondant à la formule

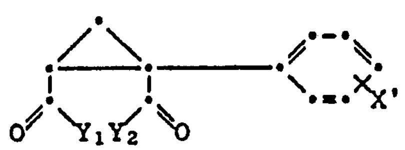

(VI),

dans laquelle $Y_1$ et $Y_2$ sont des groupes partants et X' est un groupe nitroso, hydroxyamino, carboxyle ou amino protégé, ou un sel de ceux-ci par réaction avec un composé fournissant le groupe $-NH-R_1$ et en ce qu'on

transforme dans le composé pouvant être obtenu le groupe carboxyle X' en un groupe carbamoyle ou azidocarbonyle et/ou on transforme si on le désire un composé répondant à la formule II pouvant être obtenu conformément à l'invention en un autre composé répondant à la formule II conforme à l'invention et/ou on transforme un sel pouvant être obtenu en le composé libre ou en un autre sel et/ou on transforme un composé libre pouvant être obtenu en un sel et/ou on sépare un mélange d'isomères pouvant être obtenu en les divers isomères.

22. Composés répondant à la formule

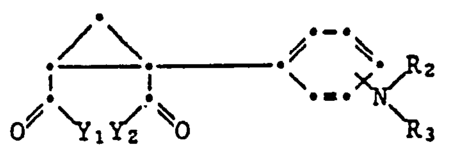

(III),

dans laquelle $R_2$ désigne un atome d'hydrogène, un groupe alkyle en $C_1$-$C_7$, sulfo, alcanoyle en $C_1$-$C_7$ ou alcansulfonyle en $C_1$-$C_7$, et $Y_1$ désigne un atome d'hydrogène ou un groupe alkyle en $C_1$-$C_7$, et $Y_1$ et $Y_2$ constituent des groupes partants, et des sels de ces composés avec des groupes formant des sels.

23. Procédé de préparation de composés répondant à la formule III selon la revendication 22, et de sels de ces composés, caractérisé en ce qu'on fait réagir un composé répondant à la formule

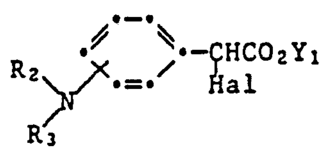

(VII),

dans laquelle $R_2$, $R_3$ et $Y_1$ ont les significations indiquées dans la revendication 22 et Hal désigne un atome de chlore ou de brome, avec un dérivé de l'acide acrylique répondant à la formule

$HC{=}CHCO_2Y_2$ (VIII),

dans laquelle $Y_2$ a la signification indiquée dans la revendication 22, et on transforme, le cas échéant, un sel pouvant être obtenu en le composé libre ou en un autre sel et/ou un composé libre pouvant être obtenu en un sel et/ou en ce qu'on sépare un mélange d'isomères pouvant être obtenu en les divers isomères.

24. Procédé de production de préparations pharmaceutiques contenant des composés répondant à la formule I, dans laquelle $R_1$, $R_2$ et $R_3$ ont les significations indiquées dans la revendication 15, ou de leurs sels, caractérisé en ce qu'on prépare des composés répondant à la formule I et des sels de ceux-ci par le procédé selon la revendication 19, et en ce qu'on les mélange avec un support pharmaceutique.

**Revendications** pour l'Etat contractant: AT

1. Procédé de préparation de composés répondant à la formule

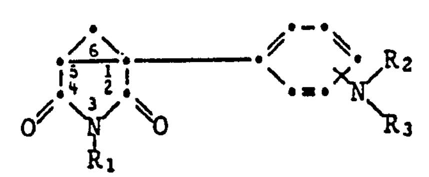

(I),

dans laquelle $R_1$ désigne un groupe alkyle en $C_1$-$C_{12}$, alcényle en $C_2$-$C_{12}$, alcinyle en $C_2$-$C_7$, cycloalkyle en $C_3$-$C_{10}$, cylcloalcényle en $C_3$-$C_{10}$, (cycloalkyle en $C_3$-$C_6$)(alkyle en $C_1$-$C_4$), (cycloalkyle en $C_3$-$C_6$)(alcényle en $C_2$-$C_4$), ou (cycloalcényle en $C_3$-$C_6$)(alkyle en $C_1$-$C_4$), $R_2$ désigne un atome d'hydrogène, un groupe alkyle en $C_1$-$C_7$, sulfo, alcanoyle en $C_1$-$C_7$ ou alcansulfonyle en $C_1$-$C_7$ et $R_3$ désigne un atome d'hydrogène ou un groupe alkyle en $C_1$-$C_7$, ou de sels de ces composés, caractérisé en ce que

a) dans un composé répondant à la formule

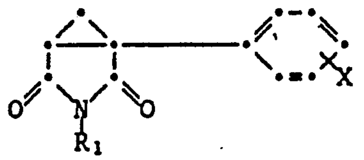 (II),

dans laquelle $R_1$ a la signification indiquée à propos de la formule I et X représente un groupe transformable en le groupe $-N(R_2)(R_3)-$, ou dans un sel de celui-ci on transforme X en le groupe $-N(R_2)(R_3)-$, ou

b) on cyclise un composé répondant à la formule

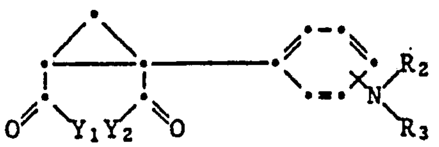 (III),

dans laquelle $R_2$ et $R_3$ ont les significations indiquées à propos de la formule I, $Y_1$ et $Y_2$ représentent des groupes partants ou un sel de celui-ci par réaction avec un composé fournissant le groupe

$$>N-R_1$$

ou

c) dans un composé répondant à la formule

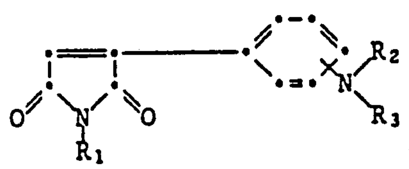 (IV),

dans laquelle $R_1$, $R_2$ et $R_3$ ont les significations indiquées à propos de la formule I, ou dans un sel de celui-ci, on fixe par addition le groupe $-CH_2$-(méthylène) sur la double liaison du maléimide, et si on le désire, on transforme un composé répondant à la formule I pouvant être obtenu conformément à l'invention en un autre composé répondant à la formule I conforme à l'invention, et/ou on transforme un sel pouvant être obtenu en le composé libre ou en un autre sel, et/ou on transforme un composé libre pouvant être obtenu en un sel et/ou on sépare un mélange d'isomères pouvant être obtenu en les divers isomères.

2. Procédé selon la revendication 1, pour la préparation de composés répondant à la formule I, dans laquelle $R_1$ désigne un groupe alkyle en $C_1$-$C_7$, $R_2$ un atome d'hydrogène, un groupe sulfo, alcanoyle en $C_1$-$C_7$ ou alcansulfonyle en $C_1$-$C_7$ et $R_3$ un atome d'hydrogène ou un groupe alkyle en $C_1$-$C_7$, et de sels de ces composés.

3. Procédé selon selon la revendication 1, pour la préparation de composés répondant à la formule I, dans laquelle $R_1$ désigne un groupe alkyle en $C_1$-$C_{12}$, alcényle en $C_2$-$C_7$, alcinyle en $C_2$-$C_7$, cycloalkyle en $C_3$-$C_{10}$ ou (cycloalkyle en $C_3$-$C_6$)(alkyle en $C_1$-$C_4$), et $R_3$ désigne un atome d'hydrogène ou un groupe alkyle en $C_1$-$C_7$, et de sels de ces composés.

4. Procédé selon la revendication 1, pour la préparation de composés répondant à la formule I, dans laquelle $R_1$ désigne un groupe alkyle en $C_1$-$C_7$ et $R_2$ et $R_3$ désignent un atome d'hydrogène ainsi que de sels de ces composés.

5. Procédé selon la revendication 1 pour la préparation de composés répondant à la formule I, dans laquelle $R_1$ désigne un groupe alkyle en $C_1$-$C_7$, alcényle en $C_2$-$C_4$, alcinyle en $C_2$-$C_4$ ou (cycloalkyle en $C_3$-$C_6$)(alkyle en $C_1$-$C_4$), le groupe $-N(R_2)(R_3)$ se trouve en position 4 du groupe phényle et $R_2$ et $R_3$ désignent un atome d'hydrogène, ainsi que de sels pharmaceutiquement acceptables de ces composés.

6. Procédé selon la revndication 1, pour la préparation de composés répondant à la formule I, dans laquelle $R_1$ désigne un groupe alkyle en $C_1$-$C_7$, le groupe $-N(R_2)(R_3)$ se trouve en position 4 du groupe phényle et $R_2$ et $R_3$ désignent un atome d'hydrogène, ainsi que de sels pharmaceutiquement acceptables de ces composés.

7. Procédé selon la revendication 1 pour la préparation de la 1-(4-aminophényl)-3-méthyl-3-azabicyclo[3.1.0]hexane-2,4-dione.

8. Procédé selon la revendication 1 pour la préparation de la 1-(4-aminophényl)-3-n-propyl-3-azabicyclo[3.1.0]hexane-2,4-dione.

9. Procédé selon la revendication 1 pour la préparation de la 1-(4-aminophényl)-3-isobutyl-3-azabicyclo[3.1.0]hexane-2,4-dione.

10. Procédé selon la revendication 1 pour la préparation de la 1-(4-aminophényl)-3-n-butyl-3-azabicyclo[3.1.0]hexane-2,4-dione.

11. Procédé selon la revendication 1 pour la préparation de la 1-(4-aminophényl)-3-n-pentyl-3-azabicyclo[3.1.0]hexane-2,4-dione.

12. Procédé selon la revendication 1, pour la préparation de la 1-(4-aminophényl)-3-néopentyl-3-azabicyclo[3.1.0]hexane-2,4-dione.

13. Procédé selon la revendication 1, pour la préparation de la 1-(4-aminophényl)-3-n-heptyl-3-azabicyclo[3.1.0]hexane-2,4-dione.

14. Procédé selon la revendication 1, pour la préparation de la 1-(4-aminophényl)-3-cyclohexylméthyl-3-azabicyclo[3.1.0] hexane-2,4-dione.

15. Procédé de préparation de composés répondant à la formule

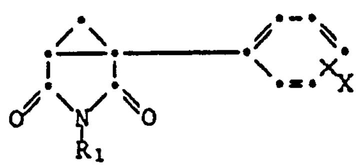

(II),

dans laquelle $R_1$ désigne un groupe alkyle en $C_1$-$C_{12}$, alcényle en $C_2$-$C_{12}$, alcinyle en $C_2$-$C_7$, cycloalkyle en $C_3$-$C_{10}$, cylcloalcényle en $C_3$-$C_{10}$, (cycloalkyle en $C_3$-$C_6$)(alkyle en $C_1$-$C_4$), (cycloalkyle en $C_3$-$C_6$)(alcényle en $C_2$-$C_4$), ou (cycloalcényle en $C_3$-$C_6$)(alkyle en $C_1$-$C_4$), et X désigne un groupe nitroso, hydroxyamino, carbamoyle, azidocarbonyle ou amino protégé, et de sels de ces composés, caractérisé en ce qu'on cyclise un composé répondant à la formule

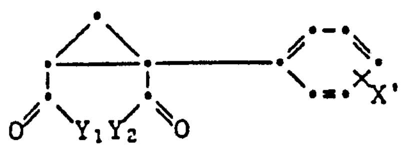

(VI),

dans laquelle $Y_1$ et $Y_2$ sont des groupes partants et X, désigne un groupe nitroso, hydroxyamino, carboxyle ou amino protégé, ou un sel de celui-ci, par réaction avec un composé fournissant le groupe -NH-$R_1$ et en ce qu'on transforme dans un composé pouvant être obtenu le groupe carboxyle X' en un groupe, carbamoyle ou azidocarbonyle et/ou on transforme, si on le désire, un composé répondant à la formule II pouvant être obtenu conformément à l'invention en un autre composé répondant à la formule II conforme à l'invention et/ou on transforme un sel pouvant être obtenu en le composé libre ou en un autre sel et/ou on transforme un composé libre pouvant être obtenu en un sel et/ou on sépare un mélange d'isomères pouvant être obtenu en les divers isomères.

16. Procédé de préparations de composés répondant à la formule

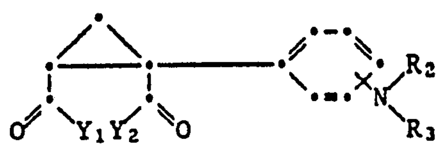

(III),

dans laquelle $R_2$ désigne un atome d'hydrogène, un groupe alkyle en $C_1$-$C_7$, sulfo, alcanoyle en $C_1$-$C_7$, ou alcansulfonyle en $C_1$-$C_7$ et $R_3$ désigne un atome d'hydrogène, ou un groupe alkyle en $C_1$-$C_7$, et $Y_1$ et $Y_2$ représentent des groupes partants et de sels de ces composés avec des groupes formant des sels, caractérisé en ce qu'on fait réagir un composé répondant à la formule

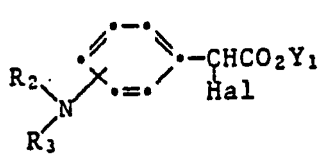

(VII),

dans laquelle $R_2$, $R_3$ et $Y_1$ ont la signification indiquée à propos de la formule III et Hal désignent un atome de chlore ou de brome avec un dérivé de l'acide acrylique répondant à la formule

$HC{=}CHCO_2Y_2$ (VIII),

dans laquelle $Y_2$ a la signification indiquée à propos de la formule III, et on transforme, si on le désire, un sel pouvant être obtenu en le composé libre ou en un autre sel et/ou on transforme un composé libre pouvant être obtenu en un sel et/ou on sépare un mélange d'isomères pouvant être obtenu en les divers isomères.

17. Procédé de production de préparations pharmaceutiques contenant des composés répondant à la formule I, dans laquelle $R_1$ désigne un atome d'hydrogène, un groupe alkyle en $C_1$-$C_{12}$, alcényle en $C_2$-$C_{12}$, alcinyle en $C_2$-$C_7$ cycloalkyle en $C_3$-$C_{10}$, cycloalcényle en $C_3$-$C_{10}$, (cycloalkyle en $C_3$-$C_6$)(alkyle en $C_1$-$C_4$), (cycloalkyle en $C_3$-$C_6$)(alcényle en $C_2$-$C_4$) ou (cylcoalcényle en $C_3$-$C_6$)(alkyle en $C_1$-$C_4$), $R_2$ désigne un atome d'hydrogène, un groupe alkyle en $C_1$-$C_7$, sulfo, alcanoyle en $C_1$-$C_7$ ou alcansulfonyle en $C_1$-$C_7$, et $R_3$ désigne un atome d'hydrogène ou un groupe alkyle en $C_1$-$C_7$, ou des sels de ceux-ci, caractérisé en ce qu'on prépare des composés répondant à la formule I et des sels de ceux-ci par le procédé selon la revendication 1, et en ce qu'on les mélange avec un support pharmaceutique.

18. Procédé selon la revendication 17, pour la production de préparations pharmaceutiques contenant de la 1-(4-aminophényl)-3-azabicyclo[3.1.0]hexane-2,4-dione.

**Revendications** pour les Etats contractants: BE et DE

1. Composés répondant à la formule

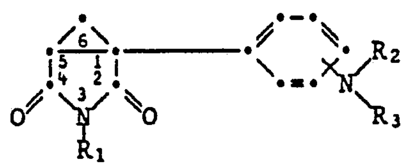

(I),

dans laquelle $R_1$ désigne un groupe alkyle en $C_1$-$C_{12}$, alcényle en $C_2$-$C_{12}$, alcinyle en $C_2$-$C_7$, cycloalkyle en $C_3$-$C_{10}$, cylcloalcényle en $C_3$-$C_{10}$, (cycloalkyle en $C_3$-$C_6$)(alkyle en $C_1$-$C_4$), (cycloalkyl en $C_3$-$C_6$)(alcényle en $C_2$-$C_4$), ou (cycloalcényle en $C_3$-$C_6$)(alkyle en $C_1$-$C_4$), $R_2$ désigne un atome d'hydrogène, un groupe alkyle en $C_1$-$C_7$, sulfo, alcanoyle en $C_1$-$C_7$ ou alcansulfonyle en $C_1$-$C_7$ et $R_3$ désigne un atome d'hydrogène ou un groupe alkyle en $C_1$-$C_7$, et les sels de ces composés.

2. Composés selon la revendication 1 répondant à la formule I, dans laquelle $R_1$ désigne un groupe alkyle en $C_1$-$C_7$, $R_2$ désigne un atome d'hydrogène, un groupe alkyle en $C_1$-$C_7$, sulfo, alcanoyle en $C_1$-$C_7$ ou alcansulfonyle en $C_1$-$C_7$ et $R_3$ désigne un atome d'hydrogène ou un groupe alkyle en $C_1$-$C_7$, et les sels de ces composés.

3. Composés selon la revendication 1, répondant à la formule I, dans laquelle $R_1$ désigne un groupe alkyle en $C_1$-$C_{12}$, alcényle en $C_2$-$C_7$, alcinyle en $C_2$-$C_7$, cycloalkyle en $C_3$-$C_{10}$ ou (cycloalkyle en $C_3$-$C_6$)(alkyle en $C_1$-$C_4$), $R_2$ désigne un atome d'hydrogène ou un groupe alkyle en $C_1$-$C_7$ et $R_3$ désigne un atome d'hydrogène ou un groupe alkyle en $C_1$-$C_7$, ainsi que leurs sels.

4. Composés selon la revendication 2, répondant à la formule I, dans laquelle $R_1$ désigne un groupe alkyle en $C_1$-$C_7$ et $R_2$ et $R_3$ désignent un atome d'hydrogène ainsi que leurs sels.

5. Composés selon la revendication 1, répondant à la formule I, dans laquelle $R_1$ désigne un groupe alkyle en $C_1$-$C_7$, alcényle en $C_2$-$C_4$, alcinyle en $C_2$-$C_4$ ou (cycloalkyle en $C_3$-$C_6$)(alkyle en $C_1$-$C_4$), le groupe -N($R_2$)($R_3$) se trouve en position 4 du groupe phényle et $R_2$ et $R_3$ désignent un atome d'hydrogène, ainsi que leurs sels pharmaceutiquement acceptables.

6. Composés selon la revendication 2, répondant à la formule I, dans laquelle $R_1$ désigne un groupe alkyle en $C_1$-$C_7$, le groupe -N($R_2$)($R_3$) se trouve en position 4 du groupe phényle et $R_2$ et $R_3$ désignent un atome d'hydrogène, ainsi que leurs sels pharmaceutiquement acceptables.

7. 1-(4-Aminophényl)-3-méthyl-3-azabicyclo[3.1.0] hexane-2,4-dione selon la revendication 1.

8. 1-(4-Aminophényl)-3-n-propyl-3-azabicyclo[3.1.0]hexane-2,4-dione selon la revendication 1.

9. 1-(4-Aminophényl)-3-isobutyl-3-azabicyclo[3.1.0]hexane-2,4-dione selon la revendication 1.

10. 1-(4-Aminophényl)-3-n-butyl-3-azabicyclo[3.1.0]hexane-2,4-dione selon la revendication 1.

11. 1-(4-Aminophényl)-3-n-pentyl-3-azabicyclo[3.1.0]hexane-2,4-dione selon la revendication 1.

12. 1-(4-Aminophényl)-3 -néopentyl-3-azabicyclo[3.1.0]hexane-2,4-dione selon la revendication 1.

13. 1-(4-Aminophényl)-3-n-heptyl-3-azabicyclo[3.1.0]hexane-2,4-dione selon la revendication 1.

14. 1-(4-Aminophényl)-3-cyclohexylméthyl-3-azabicyclo[3.1.0]hexane-2,4-dione selon la revendication 1.

15. Préparations pharmaceutiques contenant des composés répondant à la formule I, dans laquelle $R_1$ désigne un atome d'hydrogène, un groupe alkyle en $C_1$-$C_{12}$ alcényle en $C_2$-$C_{12}$, alcinyle en $C_2$-$C_7$, cycloalkyle en $C_3$-$C_{10}$, cylcloalcényle en $C_3$-$C_{10}$, (cycloalkyle en $C_3$-$C_6$)(alkyle en $C_1$-$C_4$), (cycloalkyle en $C_3$-$C_6$)(alcényle en $C_2$-$C_4$), ou (cycloalcényle en $C_3$-$C_6$) (alkyle en $C_1$-$C_4$), $R_2$ désigne un atome d'hydrogène, un groupe alkyle en $C_1$-$C_7$, sulfo, alcanoyle en $C_1$-$C_7$ ou alcansulfonyle en $C_1$-$C_7$ et $R_3$ désigne un atome d'hydrogène ou un groupe alkyle en $C_1$-$C_7$, ou des sels de ceux-ci avec un support pharmaceutiquement acceptable.

16. Préparations pharmaceutiques selon la revendication 15, contenant de la 1-(4-aminophényl)-3-azabicyclo[3.1.0]hexane-2,4-dione.

17. Composés répondant à la formule I, dans laquelle $R_1$, $R_2$ et $R_3$ ont les significations indiquées dans la revendication 15 ou leurs sels pour l'utilisation dans un procédé de traitement thérapeutique de l'organisme humain ou animal.

18. Utilisation de composés répondant à la formule I, dans laquelle $R_1$, $R_2$ et $R_3$ ont les significations indiquées dans la revendication 15, ou de leurs sels, pour la préparation d'un médicament carcinostatique.

19. Procédé de préparation de composés répondant à la formule I, selon la revendication 1, et de sels de ces composés caractérisé en ce que

a) dans un composé répondant à la formule

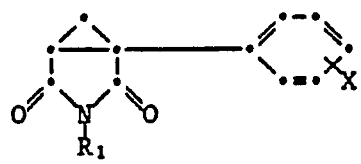

(II),

dans laquelle $R_1$ a la signification indiquée à propos de la formule I et X représente un groupe transformable en le groupe $-N(R_2)(R_3)-$, ou dans un sel de celui-ci, on transforme X en le groupe $-N(R_2)(R_3)-$, ou

b) on cyclise un composé répondant à la formule

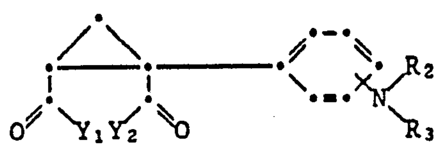

(III),

dans laquelle $R_2$ et $R_3$ ont les significations indiquées à propos de la formule I, $Y_1$ et $Y_2$ représentent des groupes partants ou un sel de celui-ci par réaction avec un composé fournissant le groupe

$$N-R_1$$

ou

c) dans un composé répondant à la formule

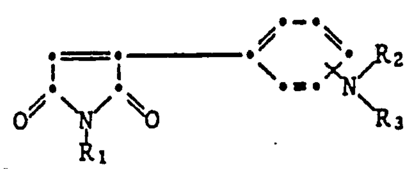

(IV),

dans laquelle $R_1$, $R_2$ et $R_3$ ont les significations indiquées à propos de la formule I, ou dans un sel de celui-ci, on fixe par addition le groupe $-CH_2-$(méthylène) sur la double liaison du maléimide, et

si on le désire, on transforme un composé répondant à la formule I pouvant être obtenu conformément à l'invention en un autre composé répondant à la formule I conforme à la définition et/ou on transforme un sel pouvant être obtenu en le composé libre ou en un autre sel, et/ou on transforme un composé libre pouvant être obtenu en un sel et/ou on sépare un mélange d'isomères pouvant être obtenu en les divers isomères.

20. Composés répondant à la formule

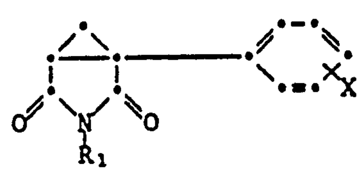

(II),

dans laquelle $R_1$ désigne un groupe alkyle en $C_1$-$C_{12}$, alcényle en $C_2$-$C_{12}$, alcinyle en $C_2$-$C_7$, cycloalkyle en $C_3$-$C_{10}$, cycloalcényle en $C_3$-$C_{10}$, (cycloalkyle en $C_3$-$C_6$)(alkyle en $C_1$-$C_4$), (cycloalkyle en $C_3$-$C_6$)(alcényle en $C_2$-$C_4$), ou (cycloalcényle en $C_3$-$C_6$)(alkyle en $C_1$-$C_4$) et X désigne un groupe nitroso, hydroxyamino, carbamoyle, azidocarbonyle ou amino protégé, et les sels de ces composés.

21. Procédé de préparation de composés répondant à la formule II selon la revendication 20, et de sels de ces composés, caractérisé en ce qu'on cyclise un composé répondant à la formule

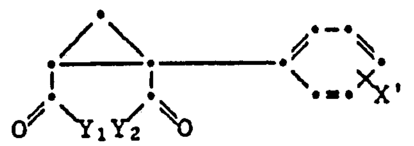 (VI),

dans laquelle $Y_1$ et $Y_2$ désignent des groupes partants et X' est un groupe nitroso, hydroxyamino, carboxyle ou amino protégé, ou un sel de ceux-ci par réaction avec un composé fournissant le groupe $-NH-R_1$ et en ce qu'on transforme dans le composé pouvant être obtenu le groupe carboxyle X' en groupe carbamoyle ou azidocarbonyle et/ou on transforme, si on le désire, un composé répondant à la formule II pouvant être obtenu conformément à l'invention en un autre composé répondant à la formule II conforme à la définition et/ou on transforme un sel pouvant être obtenu en le composé libre ou en un autre sel et/ou on transforme un composé libre pouvant être obtenu en un sel et/ou on sépare un mélange d'isomères pouvant être obtenu en les divers isomères.

22. Composés répondant à la formule

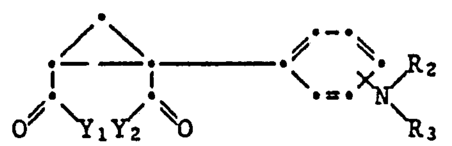 (III),

dans laquelle $R_2$ désigne un atome d'hydrogène, un groupe alkyle en $C_1$-$C_7$, sulfo, alcanoyle en $C_1$-$C_7$ ou alcansulfonyle en $C_1$-$C_7$, et $R_3$ désigne un atome d'hydrogène ou un groupe alkyle en $C_1$-$C_7$, et $Y_1$ et $Y_2$ représentent des groupes partants, et des sels de ces composés avec des groupes formant des sels.

23. Procédé de préparation de composés répondant à la formule III selon la revendication 22, et de sels de ces composés, caractérisé en ce qu'on fait réagir un composé répondant à la formule

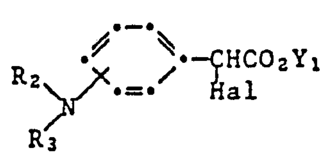 (VII),

dans laquelle $R_2$, $R_3$ et $Y_1$ ont les significations indiquées dans la revendication 22 et Hal désigne un atome de chlore ou de brome, avec un dérivé de l'acide acrylique répondant à la formule

$HC{=}CHCO_2Y_2$ (VIII),

dans laquelle $Y_2$ a la signification indiquée dans la revendication 22, et on transforme, le cas échéant, un sel pouvant être obtenu en le composé libre ou en un autre sel et/ou on transforme un composé libre pouvant être obtenu en un sel et/ou en ce qu'on sépare un mélange d'isomères pouvant être obtenu en les divers isomères.

24. Procédé de production de préparations pharmaceutiques contenant des composés répondant à la formule I, dans laquelle $R_1$, $R_2$ et $R_3$ ont les significations indiquées dans la revendication 15, ou de leurs sels, caractérisé en ce qu'on prépare des composés répondant à la formule I et des sels de ceux-ci par le procédé selon la revendication 19, et en ce qu'on les mélange avec un support pharmaceutique.